# EUROPEAN PATENT SPECIFICATION

(11) **EP 0 346 427 B1**
(45) Date of publication and mention of the grant of the patent: **29.03.1995**
(21) Application number: 89900628.2
(22) Date of filing: 02.12.1988
(51) Int. Cl.: C07H 19/01, C07D 498/18, C07D 498/22, A61K 31/70, A61K 31/33

(54) **MACROCYCLIC COMPOUNDS**
MAKROZYKLISCHE VERBINDUNGEN
COMPOSES MACROCYCLIQUES

(30) Priority: 09.12.1987 GB 8728820; 09.12.1987 GB 8728821; 13.02.1988 GB 8803370; 13.02.1988 GB 8803371; 13.02.1988 GB 8803372; 13.02.1988 GB 8803373; 13.02.1988 GB 8803374; 13.02.1988 GB 8803375; 13.02.1988 GB 8803377; 19.04.1988 GB 8809174; 23.07.1988 GB 8817624; 03.08.1988 GB 8818426
(43) Date of publication of application: 20.12.1989
(73) Proprietor: FISONS plc, Ipswich Suffolk IP1 1QH (GB)
(72) Inventor: COOPER, Martin, Edward, Leicestershire (GB); DONALD, David, Keith, Ashby de la Zouch Leicestershire (GB); HARDERN, David, Norman, Loughborough Leicestershire (GB)
(74) Representative: Wright, Robert Gordon McRae
(86) International application number: GB8801093
(87) International publication number: WO8905304

(56) References cited:
- EP-A- 184 162
- EP-A- 227 355
- EP-A- 0 349 061
- EP-A- 0 353 678

## Description

This invention relates to novel compounds, methods for their preparation, their use as medicaments, and compositions containing them.

European Patent Application 0184162 (to Fujisawa Pharmaceuticals Co. Ltd.) discloses a number of macrolides isolated form microorganisms belonging to the genus Streptomyces. The macrolides are numbered FR-900506, FR-900520, FR-900523 and FR-900525, and may be used as starting materials to produce the compounds of the present invention.

European Patent Application 353678 discloses the 3-hydroxycyclohexyl- and 3-oxocyclohexyl-analogues of FR-900506. The application establishes a prior European right in all designated states. European Patent Application 349061 discloses the 3-hydroxycyclohexyl-analogue of FR-900520. The application establishes a prior European right in Switzerland/Liechtenstein, Germany, France, United Kingdom, Italy and the Netherlands.

According to the invention, we provide compounds of formula I, 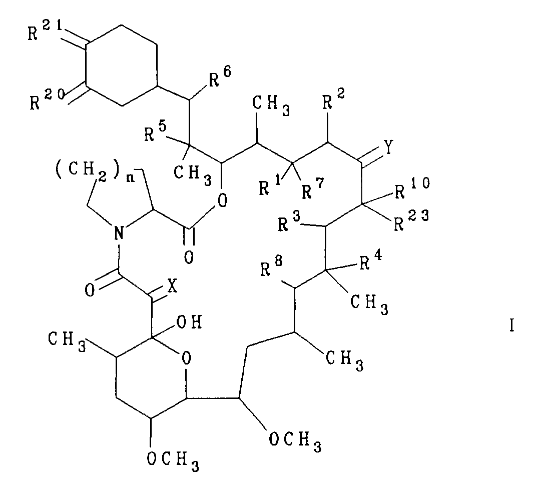
wherein each vicinal pair of substituents [R¹ and R²], [R³ and R⁴] and [R⁵ and R⁶] independently:
a) represent two vicinal hydrogen atoms, or
b) form a second bond between the vicinal carbon atoms to which they are attached;
   R⁷ represents H, OH or O-alkyl C₁₋₆, or in conjunction with R¹ it may represent =O;
   R⁸ represents H or OH;
   R¹⁰ represents alkyl C₁₋₆, alkyl C₁₋₆ substituted by one or more hydroxy groups, alkenyl C₂₋₆, alkenyl C₂₋₆ substituted by one or more hydroxy groups, or alkyl C₁₋₆ substituted by =O;
   X represents O, (H,OH) or -CH₂O-;
   Y represents O, (H,OH), N-NR¹¹R¹² or N-OR¹³;
   R¹¹ and R¹² independently represent H, alkyl C₁₋₆, phenyl or tosyl;
   R¹³ represents H or alkyl C₁₋₆;
   R²⁰ and R²¹ independently represent O, or they may independently represent (R²⁰a,H) and (R²¹a,H) respectively; R²⁰a and R²¹a independently represent OH, O-alkyl C₁₋₆ or OCH₂OCH₂CH₂OCH₃; in addition R²⁰a and R²¹a may together represent
   an oxygen atom in an epoxide ring;
   R²³ represents H;
   n is 1 or 2;
   in addition to their significances above, Y, R¹⁰ and R²³, together with the carbon atoms to which they are attached, may represent a 5- or 6-membered N-, S- or O-containing heterocyclic ring, which may be saturated or unsaturated, and which may be substituted by one or more groups selected from alkyl C₁₋₆, hydroxy, alkyl C₁₋₆ substituted by one or more hydroxy groups, O-alkyl C₁₋₆, benzyl and -CH₂Se(C₆H₅);
   provided that:
   (a) when [R³ and R⁴] and [R⁵ and R⁶] each represent a carbon-carbon bond; R⁷ represents H or an OH group; R⁸ represents H; R¹⁰ represents methyl, ethyl, propyl or allyl; R²⁰a represents OCH₃; and R²¹a represents OH; then X and Y do not both represent O; and
   (b) when [R¹ and R²] represents two vicinal hydrogen atoms; [R³ and R⁴] and [R⁵ and R⁶] each represent a carbon-carbon bond; R⁷ represents an OH group; R⁸ represents H; R¹⁰ represents allyl; R²¹a represents OH; X and Y each represent O; and n is 2; then R²⁰ does not represent O or (OH,H);
   and pharmaceutically acceptable salts thereof.

Pharmaceutically acceptable salts of the compounds of formula I include acid addition salts of any amine groups present.

Alkyl groups which R⁷, R¹¹, R¹², R¹³, R²⁰a and R²¹a may comprise include methyl.

Alkyl groups which R⁷, R¹¹, R¹², R¹³, R²⁰a and R²¹a may comprise include straight chain, branched and cyclic groups.

Alkyl groups substituted by =O which R¹⁰ may represent include ketone and aldehyde groups.

Preferably, R¹⁰ is allyl (ie. prop-2-enyl), propyl, ethyl or methyl.

Preferably, n is 2.

We prefer R⁷ to be H or OH.

Preferably, [R¹ and R²] represents two vicinal H atoms.

X is preferably O or (H,OH).

We prefer R²⁰a and R²¹a to (independently) represent OH or CH₃.

When Y, R¹⁰ and R²³ together represent a N-, S- or O- containing heterocylic ring, we prefer that ring to be five membered, more preferably a pyrrole or tetrahydrofuran ring.

According to the invention there is further provided a process for the preparation of compounds of formula I, or pharmaceutically acceptable salts thereof. The starting material for a compound of the present invention is preferably one of the macrolides isolated from microorganisms of the genus Streptomyces, which are disclosed in European Patent Application 0184162. One or more processes dicussed below may be employed to produce the desired compound of the invention.

Such a process comprises:
(a) producing a compound of formula I, in which one or more of [R¹ and R²], [R³ and R⁴] or [R⁵ and R⁶] represent two vicinal hydrogen atoms, by selective reduction of a corresponding group [R¹ and R²], [R³ and R⁴] or [R⁵ and R⁶] when it represents a second bond between two vicinal carbon atoms in a corresponding compound,
(b) producing a compound of formula I, which contains one or more hydroxy groups, by selective reduction of one or more C=O groups in a corresponding compound,
(c) producing a compound of formula I, which contains a 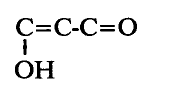 group, by selective oxidation of a 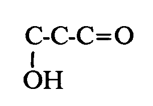 group
   in a corresponding compound,
(d) producing a compound of formula I, which contains one or more alkoxy groups, by alkylation of one or more hydroxy groups in a corresponding compound by reaction with a suitable alkylating agent,
(e) producing a compound of formula I, which contains one or more hydroxyl groups, by deprotection of one or more protected hydroxy groups in a corresponding compound where the protecting group is preferably removable by hydrogenolysis,
(f) producing a compound of formula I, which contains a carbon-carbon double bond, by elimination of HL from a corresponding compound, where L is a leaving group,
(g) producing a compound of formula I, in which Y, R¹⁰ and R²³, together with the carbon atoms to which they are attached, form a tetrahydrofuran ring substituted by a CH₂Se(C₆H₅) group by reacting a phenylselenyl halide with a corresponding compound in which Y is O and R¹⁰ is allyl,
(h) producing a compound of formula I, which contains an allylic alcohol, by selective oxidation of an allyl group in a corresponding compound,
(i) producing a compound of formula I, which contains a ketone group, by oxidation of a hydroxy group in a corresponding compound,
(j) producing a compound of formula I, which contains a vicinal diol, by oxidation of a carbon-carbon double bond in a corresponding compound,
(k) producing a compound of formula I, which contains an aldehyde group, by oxidative cleavage of a vicinal diol in a corresponding compound,
(l) producing a compound of formula I, in which Y, R¹⁰ and R²³ together with the carbon atoms to which they are attached, form a pyrrole ring, by reacting ammonia or an amine with a corresponding compound in which Y is O and R¹⁰ is -CH₂CHO,
(m) producing a compound of formula I, which contains an epoxide group, by cyclization of an 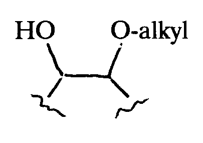 group in a corresponding compound,
(n) producing a compound of formula I, in which Y represents an oxime group, by reaction of a corresponding compound in which Y is O with an oxygen-substituted amine,
(o) producing a compound of formula I, which contains a COCH₃ group, by oxidation of a terminal alkene group in a corresponding compound,
(p) producing a compound of formula I, in which X represents -CH₂O-, by reacting a corresponding compound in which X is O with diazomethane, or
(q) producing a compound of formula I, in which Y is a hydrazone or a hydrazone derivative, by reacting a corresponding compound in which Y is O with hydrazine or a substituted hydrazine.

In process (a), reduction may be carried out catalytically using hydrogen. Suitable catalysts include platinum catalysts (eg platinum black, platinum oxides), palladium catalysts (eg palladium oxide, palladium on charcoal), nickel catalysts (eg. nickel oxide, Raney nickel), and rhodium catalysts (eg rhodium on alumina). Suitable solvents are those which do not adversely affect the reaction, and include methanol, ethanol, ethyl acetate, dichloromethane and dimethylformamide. The reduction may be carried out at or around room temperature.

Reduction may also be achieved by other means. For example, when the carbon-carbon double bond is conjugated with a ketone group, the reduction may be effected using an alkyl tin hydride, for example tri n-butyl tin hydride, in the presence of a catalyst, for example tetrakis(triphenylphosphine) palladium (0) . In this case, the reaction is preferably carried out in a solvent which does not adversely affect the reaction, for example toluene or benzene, and preferably under slightly acidic conditions, for example in the presence of a trace of acetic acid.

In process (b), suitable reagents include sodium borohydride, zinc in acetic acid, sodium triacetoxyborohydride in acetic acid, L-Selectride (Registered Trade Mark) in tetrahydrofuran, or preferably borane/^{t}butylamine complex in a solvent such as methanol or ethanol. The reduction may be conducted at or around room temperature.

In process (c), suitable oxidizing agents include dialkyl sulphoxides (eg dimethyl-sulphoxide, methylethyl-sulphoxide). The oxidation may be carried out in a solvent which does not adversely affect the reaction (eg acetone, dichloromethane, tetrahydrofuran) in the presence of an alkanoic anhydride. We prefer the anhydride to be acetic anhydride, as this may also function as the solvent for the reaction. The reaction may be conducted at or around room temperature.

In process (d), suitable alkylating agents include alkyl tosylates, diazoalkanes and alkyl halides (eg alkyl chlorides, bromides and iodides). Suitable solvents include those which are inert under the reaction conditions. We prefer polar, aprotic solvents such as dimethylformamide, 1,4-dioxan and acetonitrile. When the alkylating agent is an alkyl halide, the reaction is preferably carried out in the presence of a base, eg potassium carbonate, at a temperature of from about 0 to 100°C.

In process (e), when the hydroxyl protecting group is hydrogenolysable, hydrogenolysis may be carried out in a solvent which is inert to the reaction conditions, eg in an alcoholic solvent such as ethanol or methanol. Hydrogenolysable hydroxyl protecting groups include arylmethyl groups, in particular substituted and unsubstituted phenylmethyl groups. The reaction is preferably carried out using hydrogen at a pressure of from about 1 to 3 atmospheres using a metal catalyst on a support, eg palladium on charcoal. The hydrogenolysis is preferably carried out at a temperature of from about 0 to 50°C.

In process (f), L may be halogen or hydroxy for example.

When the precursor compound contains a 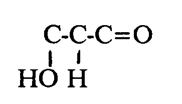
group, the elimination of H₂O may be carried out in a solvent which is inert under the reaction conditions (eg toluene) with a trace amount of acid (eg tosic acid) , at a temperature from about 50 to 100^{o}C.

In process (g), the reaction may be conducted by reacting a corresponding compound in which Y is O and R¹⁰ is allyl, with phenylselenylbromide, using methanol as solvent, at a temperature below 0^{o}C, preferably from -20 to -80^{o}C.

In process (h), suitable oxidizing agents include selenium dioxide (when other oxidizable goups are either absent or protected), preferably in the presence of ^{t}butyl hydrogen peroxide. Suitable solvents include dichloromethane, and the reaction is preferably conducted at a temperature of from 0 to 50^{o}C, more preferably 15-25^{o}C.

In process (i), suitable reagents include acidified sodium dichromate and aluminium t-butoxide (the Oppenauer method). Suitable solvents include acetone in each case; but with sodium dichromate we prefer the solvent to be acetic acid; and with aluminium t-butoxide, benzene or toluene may be added as a co-solvent. Sodium dichromate is preferably used at or around room temperature, while aluminium t-butoxide is preferably used at the reflux temperature of the reaction mixture.

In process (j), suitable reagents include osmium tetroxide, potassium permanganate, and iodine in conjunction with silver acetate. Osmium tetroxide is preferably used with a regenerating agent such as hydrogen peroxide, alkaline t-butyl hydroperoxide or N-methylmorpholine-N-oxide, and a solvent which does not adversely affect the reaction, for example diethyl ether or tetrahydrofuran. Potassium permanganate is preferably used in mild conditions, for example alkaline aqueous solution or suspensions. Co-solvents such as t-butanol or acetic acid may also be used.

Iodine-silver acetate under 'wet' conditions yields cis-diols. Preferably, iodine is used in aqueous acetic acid in the presence of silver acetate. Iodine-silver acetate under 'dry' conditions yields trans-diols. Here, the initial reaction is carried out in the absence of water, and final hydrolysis yields the diol (Prevost reaction). In each case, the oxidation is preferably carried out at a temperature from 0 to 100^{o}C, more preferably at or around room temperature.

In process (k), suitable reagents include lead tetraacetate, phenyliodoso acetate, periodic acid or sodium metaperiodate. Suitable solvents for the first two reagents include benzene and glacial acetic acid. The second two reagents are preferably used in aqueous solution. The reaction is preferably carried out at a temperature of from 0 to 100^{o}C, more preferably at or around room temperature.

In process (1) a pyrrole ring in which the nitrogen atom is unsubstituted may be produced by reacting a corresponding compound in which Y is O and R¹⁰ is -CH₂CHO with ammonia. Pyrrole rings in which the nitrogen atom is substituted may be produced by reacting the precursor compound with a substituted amine, for example 2-aminoethanol or benzylamine. Suitable solvents include those which do not adversely affect the reaction, for example dichloromethane. The reaction is preferably carried out at a temperature of from 0 to 100^{o}C, more preferably at or around room temperature.

In process (m), suitable reagents include boron trifluoride followed by diazomethane. Suitable solvents are those which do not adversely affect the reaction, for example dichloromethane. The reaction is preferably carried out at a temperature of from 0 to 100^{o}C, more preferably at or around room temperature.

In process (n), suitable oxygen-substituted amines include hydroxyl amine and O-alkyl hydroxyl-amines, for example O-methyl hydroxylamine. Suitable solvents include those which do not adversely affect the reaction, for example ethanol or methanol. The reaction is preferably carried out at a temperature of from 50-200^{o}C, more preferably at the reflux temperature of the solvent.

In process (o), suitable reagents include a palladium (II) halide, for example palladium (II) chloride, in conjunction with a cuprous halide, for example cuprous chloride. Suitable solvents include those that do not adversely affect the reaction, for example dimethyl formamide and water. The reaction is preferably carried out at a temperature of from 0 to 100^{o}C, more preferably at or around room temperature.

In process (p), suitable solvents include those which do not adversely affect the reaction, for example dichloromethane. the reaction is preferably carried out at a temperature of from 0 to 50^{o}C, more preferably at or around room temperature.

In process (q), suitable reagents include hydrazine and toluene-4-sulphonylhydrazide. Suitable solvents include those which do not adversely affect the reaction conditions, for example methanol or ethanol. The reaction is preferably carried out at a temperature of from 0 to 50^{o}C, more preferably at or around room temperature.

D. Askin et al (Tetrahedron Letts; 1988, 29, 277), S. Mills et al (ibid; 1988, 29, 281) and D Donald et al (ibid; 1988, 29, 4481) have recently disclosed synthetic routes to fragments of macrolide FR-900506 mentioned above. Their approaches may be incorporated into a process for producing the novel compounds of the present invention.

The processes described above may produce the compound of formula I or a salt thereof. It is also within the scope of this invention to treat any salt so produced to liberate the free compound of formula I, or to convert one salt into another.

The compounds of formula I, and pharmaceutically acceptable salts thereof, are useful because they possess pharmacological activity in animals; in particular they are useful because they possess immunosuppressive activity, eg in the tests set out in Examples A, B and C. Thus the compounds are indicated for use in the treatment or prevention of the resistance by transplantion of organs or tissues, such as kidney, heart, lung, bone marrow, skin, etc, and of autoimmune and proliferative diseases such as rheumatoid arthritis, systemic lupus erythematosus, Hashimoto's thyroiditis, multiple sclerosis, myasthenia gravis, type 1 diabetes, uveitis, psoriasis, etc. Some of the compounds of the invention are also indicated for use as antimicrobial agents, and thus may be used in the treatment of diseases caused by pathogenic microorganisms and the like.

We therefore provide the use of compounds of formula I (and pharmaceutically acceptable salts thereof) as pharmaceuticals.

Further, we provide the use of a compound of formula I (and pharmaceutically acceptable salts thereof) in the manufacture of a medicament for use as an immunosuppressive agent.

For the above-mentioned therapeutic uses the dosage administered will, of course, vary with the compound employed, the mode of administration, the treatment desired (eg topical, parenteral or oral) and the disease indicated. However, in general, satisfactory results are obtained when the compounds are administered at a dosage of from 0.1 to 200mg per kg of animal body weight. For man the indicated total daily dosage is in the range of from 1mg to 1000mg and preferably from 10mg to 500mg, which may be administered, for example twice weekly, or in divided doses from 1 to 6 times a day or in sustained release form. Thus unit dosage forms suitable for administration, eg oesophageally, comprise from 2mg to 500mg, and preferably 1mg to 500mg of the compound preferably admixed with a solid or liquid pharmaceutically acceptable diluent, carrier or adjuvant.

According to our invention we also provide a pharmaceutical composition comprising (preferably less than 80%, and more preferably less than 50% by weight) of a compound of formula I, or a pharmaceutically acceptable salt thereof, in combination with a pharmaceutically acceptable adjuvant, diluent or carrier. Examples of suitable adjuvants, diluents or carriers are: for tablets, capsules and dragees; microcyrstalline cellulose, calcium phosphate, diatomaceous earth, a sugar such as lactose, dextrose or mannitol, talc, stearic acid, starch, sodium bicarbonate and/or gelatin; for suppositories, natural or hardened oils or waxes; and for inhalation compositions, coarse lactose. The compound of formula I, or the pharmaceutically acceptable salt thereof, preferably is in a form having a mass median diameter of from 0.01 to 10 microns. The compositions may also contain suitable preserving, stabilising and wetting agents, solubilisers, sweetening and colouring agents and flavourings. The compositions may, if desired, be formulated in sustained release form. We prefer compositions which are designed to be taken oesophageally and to release their contents in the gastrointestinal tract.

The compounds of formula I, and pharmaceutically acceptable salts thereof, have the advantage that they are less toxic, more efficacious, are longer acting, have a broader range of activity, are more potent, produce fever side effects, are more easily absorbed or have other useful pharmacological properties, than compounds previously used in the therapeutic fields mentioned above.

The compounds of formula I have a number of chiral centres and may exist in a variety of stereoisomers. The invention provides all optical and stereoisomers, as well as racemic mixtures. The isomers may be resolved or separated by conventional techniques.

### Example A

### Mixed Lymphocyte Reaction (MLR) I

The MLR test was performed in microtitre plates, with each well containing 5 x 10⁵ C57BL/6 responder cells (H-2^{b}), 5 x 10⁵ mitomycin C treated (25ug/ml mitomycin C at 37^{o}C for 30 minutes and washed three times with RPMI 1640 medium) BALB/C stimulator cells (H-2^{d}) in 0.2ml RPMI 1640 medium supplemented with 10% fetal calf serum, 2mM sodium hydrogen carbonate, penicillin (50 unit/ml) and streptomycin (50ug/ml). The cells were incubated at 37^{o}C in a humidified atmosphere of 5% carbon dioxide and 95% of air for 68 hours and pulsed with ³H-thymidine (0.5uCI) 4 hours before the cells were collected. The object compound of this invention was dissolved in ethanol and further diluted in RPMI 1640 medium and added to the cultures to give final concentrations of 0.1ug/ml or less.

### Example B

### Mixed Lymphoctye Reaction (MLR) II

The MLR test was performed in 96-well microtitre plates with each well containing 3 x 10⁵ cells from each of two responding donors in a final volume of 0.2ml RPMI 1640 medium supplemented with 10% human serum, L-glutamine and penicillin/streptomycin. The compound under test was dissolved at 10mg/ml in ethanol and further diluted in RPMI 1640. The cells were incubated at 37^{o}C in a humidified atmosphere of 5% carbon dioxide for 96 hours. ³H-thymidine (0.5uCi) was added for the final 24 hours of the incubation to provide a measure of proliferation.

### Example C

### Graft versus Host Assay (GVH)

Spleen cells from DA and DAxLewis F1 hybrid rats were prepared at approximately 10⁸ cells/ml. 0.1ml of these suspensions were injected into the rear footpads of DAxLewis F1 rats (left and right respectively). Recipient animals were dosed with the compound under test, either orally or subcutaneously, on days 0-4. The assay is terminated on day 7 when the popliteal lymph nodes of the animals are removed and weighed. The increase in weight of the left node relative to the weight of the right is a measure of the GVH response.

The invention is illustrated by the following Examples.

### Example 1

### 17-Allyl-1-hydroxy-12-[2-(3,4-dimethoxycyclohexyl)-1-methylvinyl]-14,23,25-trimethoxy-13,19,21,27-tetramethyl-11,28-dioxa-4-azatricyclo[22.3.1.0^{4,9}]octacos-18-ene-2,3,10,16-tetraone.

To a stirred solution of the macrolide FR 900506 (200mg) in dichloromethane (30ml) and ether (20ml) was added boron trifluoride diethyl ether complex (10mg) and then a solution of diazomethane (600mg) in ether (30ml) added slowly over 5 minutes with evolution of nitrogen. The products were purified by chromatography on silica, with ether as the eluant, to yield the title compound (55mg), characterised by nmr and mass spectroscopy.
MS: (FAB) 831 (molecular ion)
¹³C NMR δ : 210.0 (C16); 196.5(C2); 166.9 (C10); 164.6 (C3); 138.5 (C19); 135.6 (C41); 133.9 (C29); 131.5 (C31); 123.3 (C18); 116.5 (C42); 97.6 (C1); 83.1 (C34); 82.6 (C35); 79.0 (C14)

### Example 2

### 17-Allyl-1,14-dihydroxy-12-[2-(3,4-dimethoxycyclohexyl) -1-methylvinyl]-23,25-dimethoxy-13,19,21,27-tetramethyl-11, 28-dioxa-4-azatricyclo[22.3.1.0^{4,9}]octacos-18-ene-2,3,10, 16-tetraone

To a stirred solution of macrolide FR 900506 (80mg) in dichloromethane (100ml) and ether (50ml) was added 35-70 micron silica (5g) and then diazomethane (300mg) in ether (50ml) was added slowly over 5 minutes with evolution of nitrogen. After stirring for a further 15 minutes the products were purified by chromatography on silica, with ether as eluant, to yield the title compound (15mg), characterised by nmr and mass spectroscopy.
MS: (FAB) 840.8 (MI+Na)
¹³C NMR δ : 212.8 (C16); 196.2 (C2); 169.0 (C.10); 164.7 (C3); 139.0 (C19); 135.6 (C41); 132.4 (C29); 129.7 (C31); 122.4 (C18); 116.7 (C42); 97.0 (C1); 83.2 (C34); 82.6 (C35)

### Example 3

### 1,14-Dihydroxy-12-[2-(4-hydroxy-3-methoxycyclohexyl)-1-methylvinyl]-18-[(phenylseleno)methyl]-16,26,28-trimethoxy-13,22,24,30-tetramethyl-11,17,31-trioxa-4-azatetracyclo [25.3.1.0^{4,9}.0^{16,20}]hentriacont-21-ene-2,3,10-trione

To a solution of the macrolide FR 900506 (62mg, 7.7x10⁻⁵moles) in dry methanol (12ml) at -78^{o}C under nitrogen was added 2,6-dimethylpyridine (9.9mg, 9.24x10⁻⁵moles). To this was then added a solution of 0.46mg of phenylselenylbromide in acetonitrile (0.47ml of a solution of 0.46mg of phenylselenylbromide in 1ml of acetonitrile), followed after 20 minutes by 0.53ml of the same solution. The reaction mixture was then evaporated at low temperature in vacuo and the residue was chromatographed on silica eluting with dichloromethane/ethyl acetate (2:1) to give the product as a mixture of diastereoisomers (65.5mg) which could be further separated by HPLC.
MS: (FAB) 1013 (MI+Na).
¹³C NMR δ : 133.2, 129.2, 127.4 (Ar); 111.6 (C10); 97.5 (C1); 78.0 (C14); 76.7 (C41); 55.8 (C9); 50.3, 49.5 (C10 rotamers); 49.8 (C17); 41.2 (C15); 31.2 (C42); 29.7 (C40).

### Example 4

### 17-Allyl-1-hydroxy-12-[2-(3,4-dimethoxycyclohexyl)-1-methylvinyl]-23,25-dimethoxy-13,19,21,27-tetramethyl-11-28-dioxa-4-azatricyclo[22.3.1.0^{4,9}]octacosa-14,18-diene-2,3,10,16-tetraone

A stirred solution of 17-Allyl-1,14-dihydroxy-12-[2-(3,4-dimethoxycyclohexyl)-1-methylvinyl]-23,25-dimethoxy-13,19,21,27-tetramethyl-11,28-dioxa-4-azatricyclo [22.3.1.0^{4,9}]octacos-18-ene-2,3,10,16-tetraone (10mg, prepared by methylation of macrolide FR 900506), in toluene (5ml) containing a trace of tosic acid was heated on a steam bath for 5 minutes. Removal of solvent in vacuo and chromatography on silica eluting with ethyl acetate gave the title compound as an oil (8mg).
MS: (FAB) 822.8 (MI+Na) 800.9 (MI+H)
¹³C NMR δ : 200.4 (C16); 192.2 (C2); 169.2 (C10); 165.0 (C3); 148.2 (C14); 138.3 (C39); 135.4 (C41); 131.4, 130.0, 127.6 (C15, C29, C31); 124.1 (C18); 116.5 (C42); 97.9 (C1); 83.3 (C34); 83.0 (C35); 79.8 (C12).

### Example 5

### 17-Allyl-1,2,14-trihydroxy-12-[2-(4-hydroxy-3-methoxy cyclohexyl)-1-methylvinyl]-23,25-dimethoxy-13,19,21,27-tetra methyl-11,28-dioxa-4-azatricyclo[22.3.1.0^{4,9}]octacos-18-ene-3,10,16-trione

To a solution of the macrolide FR 900506 (100mg) in methanol (5ml) was added a solution of Borane ^{t}Butylamine complex (3.7mg) in methanol (1ml) and the solution was stirred for 12 hours at room temperature. The solution was evaporated and chromatographed on silica using ethyl acetate as eluent to give the title compound (32mg) as a mixture of diastereoisomers.
MS: (FAB) 829 (MI+Na)
¹³C NMR (showing a mixture of rotamers) δ : 212.0, 213.4 (C16); 171, 172.8 (C10); 170.4, 169.8 (C3); 140, 140.5 (C19); 135.5, 135.6 (C41); 132.4, 132.6 (C29); 129,130 (C31); 122.5 (C18); 116.5 (C42); 99.2, 97.5 (C1).

### Example 6

### 17-Allyl-1,2,14,16-tetrahydroxy-12-[2-(4-hydroxy-3-methoxycyclohexyl)-1-methylvinyl]-23,25-dimethoxy-13,19,21, 27-tetramethyl-11,28-dioxa-4-azatricyclo[22.3.1.0^{4,9}] octacos-18-ene-3,10-dione

To a solution of the macrolide FR 900506 (40mg) in diethyl ether (5ml) was added excess borane ammonia complex (100mg) and the solution was stirred at room temperature for 1 hour. Dilute hydrochloric acid was added and the organic phase was separated and chromatographed on silica using ethyl acetate as eluant to give the title compound (25mg) as a white solid.
MS: (FAB) 830.8 (MI+Na)
¹³C NMR δ : 174.3 (C10); 171.7 (C3): 116.1 (C42).
Mp 130-150^{o}C.

### Example 7

### 17-Propyl-1,14-dihydroxy-12-[2-(4-hydroxy-3-methoxy cyclohexyl)-1-methylvinyl]-23,25-dimethoxy-13,19,21,27-tetramethyl-11,28-dioxa-4-azatricyclo[22.3.1.0.^{4,9}] octacosane-2,3,10,16-tetraone

To a solution of the macrolide FR 900506 (100mg) in methanol was added Pd-on-carbon (20mg) and the mixture was stirred in an atmosphere of hydrogen for 20 hours. Filtration of the reaction mixture, evaporation of the solvent in vacuo and HPLC of the resulting mixture on silica gave the title product (35mg).

### Example 8

### 17-Propyl-1,14-dihydroxy-12-[2-(4-hydroxy-3-methoxy cyclohexyl)-1-methylethyl]-23,25-dimethoxy-13,19,21,27-tetramethyl-11,28-dioxa-4-azatricyclo[22.3.1.0.^{4,9}]octacos-18-ene-2,3,10,16-tetraone

To a solution of the macrolide FR 900506 (100mg) in methanol was added Pd on Carbon (20mg) and the mixture was stirred in an atmosphere of hydrogen for 20 hours. Filtration of the reaction mixture, evaporation of the solvent in vacuo and HPLC of the resulting mixture on silica gave the title product (30mg).

### Example 9

### 17-Propyl-1,14-dihydroxy-12-[2-(4-hydroxy-3-methoxycyclohexyl)-1-methylethyl]-23,25-dimethoxy-13,19,21,27-tetramethyl-11,28-dioxa-4-azatricyclo[22.3.1.0^{4,9}]octacosane-2,3,10,16-tetraone

To a solution of the macrolide FR 900506 (100mg) in methanol was added Pd on Carbon (20mg) and the mixture was stirred in an atmosphere of hydrogen for 20 hours. Filtration of the reaction mixture, evaporation of the solvent in vacuo and HPLC of the resulting mixture on silica gave the title product (15mg).

### Example 10 (Illustrative)

### 17-Propyl-1-14-dihydroxy-12-[2-(4-hydroxy-3-methoxycyclohexyl-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone

To a solution of the macrolide FR 900506 (800mg) in ethanol (20ml) was added Pd-on-carbon (10mg) and the mixture was stirred in an atmosphere of hydrogen for 30 minutes. Filtration of the reaction mixture, evaporation of the solvent in vacuo and chromatography on silica eluting with ether/methanol (20:1) yielded the title compound as an oil (750mg).
¹³C NMR δ : 33.32 (C40); 20.43 (C41) ; 14.11 (C42)

### Example 11 (Illustrative)

### 17-Propyl-l-hydroxy-12-[2-(4-hydroxy-3-methoxycyclohexyl)-l-methylvinyl-23,25-dimethoxy-13,19,21,27-tetramethyl-11,28-dioxa-4-azatricyclo[22.3.1.0^{4,9}]octacosa-14,18-diene-2,3,10,16-tetraone

A stirred solution of 17-propyl-1,14-dihydroxy-12-[2-(4-hydroxy-3-methoxycyclohexyl)-l-methylvinyl-23,25-dimethoxy-13,19,21,27-tetramethyl-11,28-dioxa-4-azatricyclo[22.3.1.0^{4,9}]octacos-18-ene-2,3,10,16-tetraone (800mg prepared by the method of example 10), in toluene (20ml) containing 50mg of tosic acid was heated on a steam bath for 30 minutes. Removal of solvent in vacuo and chromatography on silica eluting with ether gave the title compound as an oil (600mg).
MS : (FAB) 811 (molecular ion + Na)
¹³C NMR δ : 34.64 (C40); 20.54 (C41); 14.08 (C42); 201.21 (major), 199.76 (minor) (C16); 147.93 (major), 146.25 (minor).

### Example 12

### 17-Propyl-1-hydroxy-12-[2-(4-hydroxy-3-methoxycyclo hexyl)-1-methylvinyl]-23,25-dimethoxy-13,19,21,27-tetra methyl-11,28-dioxa-4-azatricyclo[22.3.1.0^{4,9}]octacos-18-ene-2,3,10,16-tetraone

0.75ml of tributyl tin hydride, 0.06ml of water and 150mg of (Ph₃P)₄Pd were added in three portions over a period of 1 hour to a stirred solution of the title compound of example 11 (600mg) in tetrahydrofuran (20ml) at room temperature. Water was then added, and the mixture extracted into ether. Removal of solvent in vacuo and chromatography on silica eluting with ether gave the title compound as an oil (400mg).
MS : (FAB) 813 (MI + Na)
¹³C NMR δ : 212.3 (C16); 196.4 (C2); 169.4 (C10); 165.1 (C3); 138.1 (C19); 131.7 (C31); 124.3 (C18); 97.4 (C1); 84.1 (C34); 82.4 (C12).

### Example 13

### 17-Allyl-1,14,20-trihydroxy-12-[2-(4-hydroxy-3-methoxy cyclohexyl)-1-methylvinyl]-23,25-dimethoxy-13,19,21,27-tetra methyl-11,28-dioxa-4-azatricyclo[22.3.1.0^{4,9}]octacos-18-ene-2,3,10,16-tetraone and

### 17-(1-Hydroxyprop-2-enyl)-1,14,20-trihydroxy-12-[2-(4-hydroxy-3-methoxycyclohexyl)-1-methylvinyl]-23,25-dimethoxy-13,19,21,27-tetramethyl-11,28-dioxa-4-azatricyclo [22.3.1.0^{4,9}]octacos-18-ene-2,3,10,16-tetraone

To a stirred solution of the macrolide FR 900506 (140mg) in dichloromethane (17.5ml) at room temperature was added SeO₂ (700mg), followed by tertiary-butyl hydrogen peroxide (1.05ml of a 70% aqueous solution). The reaction mixture was left to stir for 60 hours, after which it was extracted with ethyl acetate. The organic phase was washed with water followed by brine, dried (MgSO₄) and concentrated in vacuo. Flash chromatography on silica eluting with ether/methanol (20:1) yielded the title compounds separately as oils (19mg and 15mg respectively).
¹³C NMR [CDCl₃] δ:
(first compound) 141.4 (C19); 123.5 (C18); 135.4 (C41); 117.0 (C42); 131.6 (C29); 129.1 (C13); 211.4 (C16); 195.4 (C2); 170.4 (C10); 166.7 (C3); 98.2 (C1) and 84.1 (C34)ppm,
(second compound) 142.3 (C19); 120.7 (C18); 137.5 (C41); 115.9 (C42); 132.3 (C29); 129.0 (C31); 210.7 (C16); 195.8 (C2); 170.5 (C10); 167.2 (C3); 98.2 (C1) and 84.1 (C34) ppm
MS: (FAB) (first compound) 904 (MI+Rb), 842 (MI+Na);
(second compound) 920 (MI+Rb), 859 (MI+Na);

### Example 14 (Illustrative)

### 17-Allyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacosa-14,18-diene-2,3,10,16-tetraone

A stirred solution of the macrolide FR 900506 (200mg) in dry toluene (10ml) containing 5mg of tosic acid was heated on a steam bath for 30 minutes. Removal of solvent in vacuo and chromatography on silica eluting with ether gave the title compound as an oil (160mg).
MS: (FAB) 808 (MI+Na); 786 (MI+H).
¹³C NMR δ : 200.4 (C16); 196.0 (C2); 169.3 (C10); 165.0 (C3); 148.0 (C14); 138.3 (C39); 135.5 (C41); 123.4 (C18); 116.6 (C42); 98.0 (C1); 84.2 (C34); 79.8 (C12).

### Example 15

### 17-Allyl-1,2-dihydroxy-12-[2-(4-hydroxy-3-methoxy-cyclohexyl)-1-methylvinyl]-23,25-dimethoxy-13,19,21,27-tetra methyl-11,28-dioxa-4-azatricyclo-[22.3.1.0^{4,9}]octacos-18-ene-3,10,16-trione

To a stirred solution of the title compound of example 14 (60mg) in glacial acetic acid (5ml) was added powdered zinc (1g). Stirring was continued for 1 hour when the reaction was complete. The reaction mixture was then extracted with ethyl acetate, washed with saturated NaHCO₃ solution followed by brine, dried (MgSO₄) and concentrated in vacuo. Chromatography on silica eluting with ether/methanol (15:1 then 10:1) gave the title compound as an oil (30mg).
¹³C NMR[CDCl₃] δ : 99.1(C1); 67.9(C2); 171.2 and 171.7(C10 and C3); 44.6(C5); 83.32(C12); 84.0(C34); 76.6(C23); 71.7(C24); 73.3 and 73.9(C25 and C35); 52.9(C9); 52.7(C17) and 49.5(C20) ppm.
MS: (FAB) 874 (MI+Rb); 813 (MI+Na).

### Example 16

### 17-Allyl-1,16-dihydroxy-12-[2-(4-hydroxy-3-methoxycyclohexyl)-1-methylvinyl]-23,25-dimethoxy-13,19,21,27-tetra methyl-11,28-dioxa-4-azatricyclo[22.3.1.0^{4,9}]octacosa-14, 18-diene-2,3,10-trione

The title compound from example 14 (50mg) was dissolved in tetrahydrofuran (3ml) and t-butanol (0.05ml). The resulting solution was added dropwise to a stirred solution of L-Selectride (Registered Trade Mark) (0.3ml of a 1M solution in tetrahydrofuran) under a nitrogen atmosphere at -78^{o}C. Stirring was continued for 40 minutes, after which saturated ammonium chloride solution (5ml) was added and the mixture extracted with ethyl acetate. After filtration of the organic phase, and removal of solvent in vacuo, chromatography on silica eluting with ether/methanol (15:1) yielded the title compound as an oil (10mg).
¹³C NMR[CDCl₃] δ : 197.0(C2); 169.1(C10); 165.3(C3); 96.4(C1) and 84.2(C34) ppm.
MS: (FAB) 872 (MI+Rb); 810 (MI+Na).

### Example 17

### 17-Allyl-l-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone

To a stirred solution of 17-allyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-14,18-diene-2,3,10,16-tetraone (as prepared in Example 14) (100mg) in toluene (5ml) and acetic acid (0.01ml) was added tetrakis(triphenylphosphine) palladium(0) (0.01g). After 5 minutes tri n-butyltin hydride (0.04g) was added and the reaction mixture was stirred at room temperature for 2 hours. Water was added and the reaction mixture was extracted with ether. The ether extracts were dried (magnesium sulphate), filtered and evaporated to an oil in vacuo. Chromatography on silica eluting with ether gave the title compound as a low melting solid (70mg).
MS: (FAB) 810.7 (MI+Na), 788.7 (MI+H).
¹³C NMR δ : 211.3 (C16); 196.3 (C2); 169.2 (C10); 164.9 (C3); 138.4 (C19); 135.5 (C41); 131.6 (C29); 130.9 (C31); 123.3 (C18); 116.3 (C42); 97.2 (C1); 84.0 (C34); 82.2 (C12).

### Example 18

### 17-Propyl-1-hydroxy-12-[2-(3-methoxy-4-oxocyclohexyl)-1 -methylvinyl]-23,25-dimethoxy-13,19,21,27-tetramethyl-11,28-dioxa-4-azatricyclo[22.3.1.0^{4,9}]octacos-18-ene-2,3,10,16-tetraone.

To a stirred solution of the title compound from Example 12 (25mg) in acetic acid (5ml) was added potassium dichromate (25mg), and stirring was continued overnight. The solution was then evaporated to dryness. Chromatography on silica using ether as eluant gave the title compound (15mg).
MS: (FAB) 810 (MI+Na); 788 (MI+H).
¹³C NMR δ : 212.2 (C35); 208.7 (C16); 196.3 (C2); 169.4 (C10; 165.2 (C3); 138.2 (C19); 132.5 (C29) 129.2 - 124.2 (C31-C42); 97.3 (C1); 83.0 (C34); 82.0 (C12)

### Example 19

### 17-(2,3-Dihydroxypropyl)-1,14-dihydroxy-12-[2-(4-hydroxy-3-methoxycyclohexyl)-1-methylvinyl]-23,25-dimethoxy-13,19,21,27,-tetramethyl-11,28-dioxa-4-azatricyclo [22.3.1.0^{4,9}]octacos-18-ene-2,3,10,16-tetraone.

A solution of macrolide 900506 (70mg), N-methylmorpholine-N-oxide (NMO) (70mg), osmium tetroxide (4mg) and water (0.1ml) in tetrahydrofuran (5ml) was stirred at room temperature for 2 hours, and then treated with powdered sodium metabisulphite (100mg) and Florisil (Registered Trade Mark). The mixture was diluted with ethyl acetate, filtered through celite, then washed with saturated NaHCO₃ solution, followed by brine. The solution was dried (MgSO₄) and concentrated in vacuo to yield the crude title compound.
MS: (FAB) 921 (MI+Rb), 861 (MI+Na).

### Example 20

### 17-Ethanalyl-1,14-dihydroxy-12-[2-(4-hydroxy-3-methoxycyclohexyl)-1-methylvinyl]-23,25-dimethoxy-13,19,21, 27-tetramethyl-11,28-dioxa-4-azatricyclo[22.3.1.0^{4,9}] octacos-18-ene-2,3,10,16,-tetraone.

The crude title compound from Example 19 was dissolved in benzene (5ml) and treated with lead tetraacetate (100mg) for 2-3 minutes at room temperature. The solution was then diluted with ethyl acetate, washed with saturated NaHCO₃ solution followed by brine, dried (MgSO₄) and concentrated in vacuo to yield the crude product.
MS: (FAB) 889 (MI+Rb), 829 (MI+Na)

### Example 21

### 1,14-dihydroxy-12-[2-(4-hydroxy-3-methoxycyclohexyl)-1-methylvinyl]-26,28-dimethoxy-13,22,24,30-tetramethyl-11,31-dioxa-4,17-diazatetracyclo[25.3.1.0^{4,9}.0^{16,20}] hentriaconta-16(20),18,21-triene-2,3,10-trione.

The crude title compound from Example 20 was dissolved in dichloromethane and treated with 0.88M NH₃ (aq) (0.2ml). After stirring for 5 minutes at room temperature the solution was diluted with ethyl acetate, washed with saturated NaHCO₃ solution, dried (MgSO₄) and concentrated in vacuo. Chromatography on silica yielded the title compound (18mg).
MS: (FAB) 872 (MI+Rb), 787 (MI).
¹³C NMR δ : 196.44 (C2); 169.67 (C10); 165.44 (C3); 97.53 (C1).

### Example 22

### 1,14-Dihydroxy-12-[2-(4-hydroxy-3-methoxycyclohexyl)-1-methylvinyl]-26,28-dimethoxy-17-(2-hydroxyethyl)-13,22,24,30 -tetramethyl-11,31-dioxa-4,17-diazatetracyclo[25.3.1.0.^{4,9} .0^{16,20}]hentriaconta-16(20),18,21-triene-2,3,10-trione.

Following the method of Example 21, the title compound (25mg) was prepared by treating the title compound of Example 20 with 2-aminoethanol (0.2ml).
MS: (FAB) 915 (MI+Rb), 831 (M+H).
¹³C NMR δ : 196.50 (C2); 169.32 (C10); 165.50 (C3); 97.15 (C1).

### Example 23

### 1,14-Dihydroxy-12-[2-(4-hydroxy-3-methoxycyclohexyl)-1 -methylvinyl]-26,28-dimethoxy-13,22,24,30-tetramethyl-17-phenylmethyl-11,31-dioxa-4,17-diazatetracyclo[25.3.1.0^{4,9}. 0^{16,20}]hentriaconta-16(20),18,21-triene-2,3,10-trione.

Following the method of Example 21, the title compound (30mg) was prepared by treating the title compound of Example 20 with benzylamine (0.1ml).
MS: (FAB) 960 (MI+Rb), 876 (MI).
¹³C NMR δ : 196.44 (C2); 169.67 (C10); 165.44 (C3); 97.53 (C1).

### Example 24

### 17-Allyl-1-hydroxy-12-[2-(3,4-epoxycyclohexyl)-1-methyl vinyl]-23,25-dimethoxy-13,19,21,27-tetramethyl-11,28-dioxa-4 -azatricyclo[22.3.1.0^{4,9}]octacosa-14,18-diene-2,3,10,16-tetraone

To a solution of the title compound of Example 14 (823mg, 1.05mmole) in dry dichloromethane (50ml) was added boron trifluoride diethyl etherate (1 drop) followed by portionwise addition of a dried solution of diazomethane in diethyl ether until no starting material remained. Sodium carbonate was then added, and the resulting mixture stirred for 30 minutes at room temperature. The reaction mixture was then filtered, concentrated in vacuo, and chromatographed on silica eluting with 40-60^{o} petroleum ether/ethyl acetate [3:1] to give the title compound as an oil (45mg).
¹³C NMR δ : 51.3 (C34/C35)
MS: (FAB) 838.64 (MI+Rb), 776.85 (MI+Na).

### Example 25

### 17-Allyl-1,14-dihydroxy-12-[2-(4-hydroxy-3-methoxycyclohexyl)-1-methylvinyl]-23,25-dimethoxy-13,19,21, 27-tetramethyl-11,28-dioxa-4-azatricyclo[22.3.1.0^{4,9}] octacos-18-ene-2,3,10,16-tetraone C16 oxime.

A mixture of macrolide 900506 (40mg), hydroxylamine hydrochloride (40mg) and pyridine (20mg) in ethanol (5ml) was heated under reflux for 3 hours. The solution was poured into dilute hydrochloric acid and extracted into dichloromethane. The organic phase was separated and chromatographed on silica, eluting with ethyl acetate to yield the title compound as a colourless solid (25mg).

A 1:1 mixture of syn and anti oximes was present.
¹³C NMR δ : 196.8 (C2); 169.0 (C10); 165.2 (C3); 162.0 (C16); 138.7 (C19); 135.9 (C41); 132.3 (C29); 129.0 (C31); 125.2 (C18); 116.0 (C42); 97.6 (C1); 84.3 (C34).
MS: (FAB) 834 (MI).

### Example 26

### 17-Allyl-1,14-dihydroxy-12-[2-(4-hydroxy-3-methoxycyclohexyl)-1-methylvinyl]-23,25-dimethoxy-13,19,21, 27-tetramethyl-11,28-dioxa-4-azatricyclo[22.3.1.0^{4,9}] octacos-18-ene-2,3,10,16-tetraone C16 oxime O-methyl ether.

A mixture of macrolide 900506 (100mg), 0-methyl hydroxylamine hydrochloride (40mg) and pyridine (50mg) in ethanol (5ml) was heated under reflux for 3 hours. The solvent was evaporated and the product chromatographed on silica eluting with ethyl acetate to give the product as a colourless solid (50mg).

A 1:1 mixture of syn and anti oximes was present.
¹³C NMR δ : 196.4 (C2); 169.1 (C10); 165.2 (C3); 160.1 (C16); 138.2 (C19); 135.8 (C41); 132.6 (C29); 128 (C31); 125 (C18); 116.2 (C42); 97.0 (C1); 84.2 (C34); 61.7 (=NOCH₃); 56.2 (C17).
MS: (FAB) 833 (MI+H)

### Example 27

### 17-Ally-1,14-dihydroxy-12-[2-(4-(2',5'-dioxahexyloxy)-3 -methoxycyclohexyl-1-methylvinyl]-23,25-dimethoxy-13,19,21, 27-tetramethyl-11,28-dioxa-4-azatricyclo[22.3.1.0^{4,9}] octacos-18-ene-2,3,10,16-tetraone.

To a solution of macrolide 900506 (100mg) in dichloromethane (2ml) was added 2-methoxyethoxymethyl (MEM) chloride (155mg) and N,N-diisopropylethylamine (160mg). After stirring for 90 minutes at room temperature, volatiles were removed in vacuo and the reaction mixture was purified by chromatography on silica eluting with 40-60^{o} petroleum ether/acetone [3:1] to give the title compound as an oil (72mg).
MS: (FAB) 915 (MI+Na)
¹³C NMR δ : 95 (C1'MEM); 71.7 and 66.7 (C3' and C4' MEM); 58.97 (C6'MEM); 30.7 (C36); 79.4 (C35); 82.6 (C34).

### Example 28

### 17-Propyl-1-hydroxy-12-[2-(3,4-dihydroxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone.

To a solution of 17-Allyl-1,14-dihydroxy-12-[2-(3,4-dihydroxycyclohexyl)-1-methylvinyl]-23,25-dimethoxy-13,19, 21,27-tetramethyl-11,28-dioxa-4-azatricyclo[22.3.1.0^{4,9}] octacos-18-ene-2,3,10,16-tetraone (100mg) in toluene (20ml) was added p-toluenesulphonic acid (5mg) and the resulting solution was warmed for 90 minutes on a steam bath. Evaporation of volatiles in vacuo and chromatography of the residue on silica eluting with ethyl acetate gave an oil which was dissolved in methanol (5ml). To this was then added 10% palladium-on-carbon (20mg) and the mixture was stirred in an atmosphere of hydrogen for 1 hour at room temperature. The reaction mixture was then filtered through celite, concentrated in vacuo and purified by chromatography on silica eluting with ethyl acetate to give the title compound as an oil (40mg).
MS: (FAB) 799 (M+Na) 861 (M+Rb)

### Example 29

### 1,14-Dihydroxy-12-[2-(4-hydroxy-3-methoxycyclohexyl)-1-methylvinyl]-23,25-dimethoxy-13,19,21,27-tetramethyl-17-(2-oxopropyl)-11,28,-dioxa-4-azatricyclo[22.3.1.0^{4,9}] octacos-18-ene-2,3,10,16-tetraone.

A solution of macrolide 900506 (250mg) in dimethylformamide (2ml) was added to a stirred mixture of cuprous chloride (150mg) and palladium (2) chloride (50mg) in dimethylformamide (6ml) and water (1.2ml) at room temperature. A slow stream of air was passed through the reaction mixture which was stirred at room temperature for 1.5 hours. The reaction mixture was then diluted with diethyl ether (200ml), washed with dilute aqueous hydrochloric acid (1Mx2) and brine, dried (magnesium sulphate), filtered and concentrated to an oil in vacuo. Chromatography on silica eluting with hexane/acetone [2:1] gave the title compound as a foam (158mg).
MS (FAB) 821 (MI+H), 843 (MI+Na), 905 (MI+Rb).
¹³C NMR δ : (major isomer) 97.11 (C1); 196.02 (C2); 164.60 (C.3); 168.74 (C10); 213.04 (C16); 120.83 (C18); 138.51 (C19); 132.71 (C29); 129.07 (C31); 29.64 (C42); 207.74 (C41).
(minor isomer) 98.29 (C1); 193.33 (C2); 165.75 (C3); 168.67 (C10); 212.90 (C16); 120.47 (C18); 140.29 (C19); 132.17 (C29); 129.29 (C31); 207.86 (C41).

### Example 30

### 17-Allyl-1,14-dihydroxy-12-[2-(4-hydroxy-3-methoxycyclohexyl)-1-methylvinyl]-23,25-dimethoxy-13,19,21, 27-tetramethyl-11,28-dioxa-4-aza-spiro[tricyclo [22.3.1.0^{4,9}]octacos-18-ene-2,2'-oxirane]-3,10,16-trione.

A solution of diazomethane (excess) in dry ether was added to a solution of macrolide 900506 (50mg) in dichloromethane (5ml). The solution was stirred for 2 hours, then chromatographed on silica using ethyl acetate as eluant. The title compound was obtained as a colourless solid.
MS: (FAB) 818 (MI).
¹³C NMR δ : 212 (C16); 170.4 (C10); 165.7 (C3); 139.0 (C19); 135.4 (C41); 132.4 (C29); 129.4 (C31); 132.0 (C18); 116.8 (C42); 96.7 (C1); 84.2 (C34); 61.6 (C2) 50.7 (C2a)

### Example 31

### 17-Ethanalyl-1,2,14-trihydroxy-12-[2-(4-hydroxy-3-methoxycyclohexyl-1-methylvinyl]-23,25-dimethoxy-13,19,21, 27-tetramethyl-11,28-dioxa-4-azatricyclo[22.3.1.0^{4,9}] octacos-18-ene-3,10,16-trione.

A sample of the crude product from Example 20 (15mg) was dissolved in acetic acid (3ml).

Zinc dust (0.5g) was then added and the mixture was stirred at room temperature for 30 minutes. After aqueous work up and column chromatography on silica the title compound was isolated as an oil (10mg).
¹³C NMR δ : (1:1 mixture of rotamers) 99.08, 97.75 (C1); 212.81, 209.84 (C16); 200.61, 200.27 (C41); 172.40, 171.25, 170.41, 169.84 (C10, C3); 141.28, 140.96 (C29); 133.06, 132.50 (C29); 130.32, 128.69 (C31); 121.07, 120.47 (C18).
MS: (FAB) 892 (MI+Rb), 831 (MI+Na).

### Example 32

### 1,14-Dihydroxy-12-[2-(4-hydroxy-3-methoxycyclohexyl)-1-methylvinyl]-26,28-dimethoxy-18-[(phenylseleno)methyl]-13, 22,24,30-tetramethyl-11,17,31-trioxa-4-azatetracyclo[25.3.1. 0.^{4,9}0.^{16,20}]hentriaconta-16(20),21-diene-2,3,10-trione.

To a cold (-78^{o}C) solution of macrolide 900506 (198.5mg) and 2,6-dimethylpyridine (29mg) in dry methanol (8ml) was added a solution of phenylselenyl bromide (127.3mg) in dry acetonitrile (2.2ml) under nitrogen. Solvents were then removed in vacuo at low temperature and the residual oil was purified by column chromatography on silica eluting with dichloromethane/ethyl acetate [2:1] to yield the title compound as an oil (37mg).
MS: (FAB) 959 (MI+H)
¹³C NMR δ : 29.72 (C19); 75.76 (C18); 106.7 (C20); 153.24 (C16).

### Example 33

### Benzenesulphonic acid, 4'-methyl-[17-Allyl-1,14-dihydroxy-12-[2-(4-hydroxy-3-methoxycyclohexyl-1-methylvinyl]-23,25-dimethoxy-13,19,21,27-tetramethyl-11,28-dioxa-4-azatricyclo[22.3.1.0^{4.9}]octacos-18-ene-2,3, 10-trione-16-ylidene]hydrazide.

To a stirred solution of macrolide 900506 (23mg) in ethanol (3ml) was added toluene-4-sulphonylhydrazide (5.33mg) and toluene-4-sulphonic acid (5.45mg). The reaction mixture was stirred overnight at room temperature. Solvents were evaporated in vacuo and the residue was purified by column chromatography on silica, eluting with diethyl ether, to yield the title compound (5mg) as an oil.
MS: (FAB) 954 (MI-OH), 972 (MI+H), 994 (MI+Na)

### Example 34

A selection of compounds were tested according to Example B. The concentration of compound required to inhibit the proliferation of lymphocytes by 50% was measured, and the results were as follows:

| Example No. of product compound | IC₅₀ (M) |
|---|---|
| 1 | < 1x10⁻⁶ |
| 2 | < 1x10⁻⁶ |
| 5 | < 1x10⁻⁶ |
| 6 | < 1x10⁻⁶ |
| 12 | < 1x10⁻⁶ |
| 15 | < 1x10⁻⁶ |
| 17 | < 1x10⁻⁶ |
| 18 | < 1x10⁻⁶ |
| 22 | < 1x10⁻⁶ |
| 25 | < 1x10⁻⁶ |
| 27 | < 1x10⁻⁶ |
| 30 | < 1x10⁻⁶ |
| 32 | < 1x10⁻⁶ |

## Claims (Claims for the following Contracting State(s): CH, LI, DE, FR, GB, IT, NL)

1. A compound of formula I, 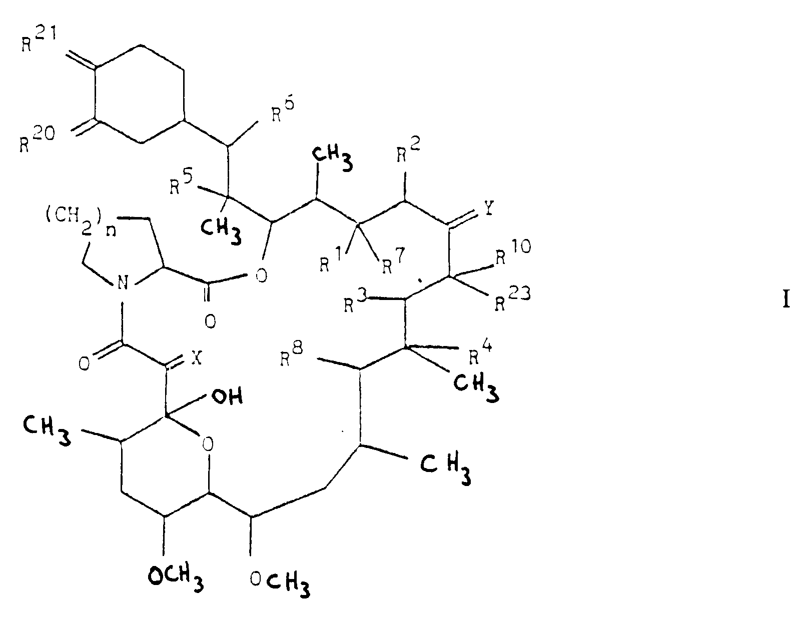 wherein each vicinal pair of substituents [R¹ and R²], [R³ and R⁴] and [R⁵ and R⁶] independently:
a) represent two vicinal hydrogen atoms, or
b) form a second bond between the vicinal carbon atoms to which they are attached;
R⁷ represents H, OH or O-alkyl C₁₋₆, or in conjunction with R¹ it may represent =O;
R⁸ represents H or OH;
R¹⁰ represents alkyl C₁₋₆, alkyl C₁₋₆ substituted by one or more hydroxy groups, alkenyl C₂₋₆, alkenyl C₂₋₆ substituted by one or more hydroxy groups, or alkyl C₁₋₆ substituted by =O;
X represents O, (H,OH) or -CH₂O-;
Y represents O, (H,OH), N-NR¹¹R¹² or N-OR¹³;
R¹¹ and R¹² independently represent H, alkyl C₁₋₆, phenyl or tosyl;
R¹³ represents H or alkyl C₁₋₆;
R²⁰ and R²¹ independently represent O, or they may independently represent (R²⁰a,H) and (R²¹a,H) respectively; R²⁰a and R²¹a independently represent OH, O-alkyl C₁₋₆ or OCH₂OCH₂CH₂OCH₃; in addition R²⁰a and R²¹a may together represent an oxygen atom in an epoxide ring;
R²³ represents H;
n is 1 or 2;
in addition to their significances above, Y, R¹⁰ and R²³, together with the carbon atoms to which they are attached, may represent a 5- or 6-membered N-, S- or O-containing heterocyclic ring, which may be saturated or unsaturated, and which may be substituted by one or more groups selected from alkyl C₁₋₆, hydroxy, alkyl C₁₋₆ substituted by one or more hydroxy groups, O-alkyl C₁₋₆, benzyl and -CH₂Se(C₆H₅);
provided that:
(a) when [R³ and R⁴] and [R⁵ and R⁶] each represent a carbon-carbon bond; R⁷ represents H or an OH group; R⁸ represents H; R¹⁰ represents methyl, ethyl, propyl or allyl; R²⁰a represents OCH₃; and R²¹a represents OH; then X and Y do not both represent O;
(b) when [R¹ and R²] represents two vicinal hydrogen atoms; [R³ and R⁴] and [R⁵ and R⁶] each represent a carbon-carbon bond; R⁷ represents an OH group; R⁸ represents H; R¹⁰ represents allyl; R²¹a represents OH; X and Y each represent O; and n is 2; then R²⁰ does not represent O or (OH,H); and
(c) when [R¹ and R²] represents two vicinal hydrogen atoms; [R³ and R⁴] and [R⁵ and R⁶] each represent a carbon-carbon bond; R⁷ represents an OH group; R⁸ represents H; R¹⁰ represents ethyl; R²¹a represents OH; X and Y each represent O; and n is 2; then R²⁰a does not represent OH;
and pharmaceutically acceptable salts thereof.

2. A compound according to claim 1, wherein R¹⁰ represents methyl, ethyl, propyl or allyl.

3. A compound according to claim 1 or claim 2, wherein at least one of R²⁰a and R²¹a represents OH or OCH₃.

4. A compound according to any one of the preceding claims, wherein n is 2.

5. A compound according to any one of the preceding claims, wherein R⁷ represents H or OH.

6. A compound according to claim 1, which is:
17-Allyl-1-hydroxy-12-[2-(3,4-dimethoxycyclohexy)-1-methylvinyl]-14,23,25-trimethoxy-13,19,21,27-tetramethyl-11,28-dioxa-4-azatricyclo[22.3.1.0^{4,9}]octacos-18-ene-2,3,10,16-tetraone,
17-Allyl-1,14-dihydroxy-12-[2-(3,4-dimethoxycyclohexyl)-1-methylvinyl]-23,25-dimethoxy -13,19,21,27-tetramethyl-11,28-dioxa-4-azatricyclo[22.3.1.0^{4,9}]octacos-18-ene-2,3,10,16-tetraone,
1,14-Dihydroxy-12-[2-(4-hydroxy-3-methoxycyclohexyl)-1-methylvinyl]-18-[(phenylseleno)methyl]-16,26,28-trimethoxy-13,22,24,30-tetramethyl-11,17,31-trioxa-4-azatetracyclo[25.3.1.0^{4,9}.0^{16,20}]hentriacont-21-ene-2,3,10-trione,
17-Allyl-1-hydroxy-12-[2-(3,4-dimethoxycyclohexy)-1-methylvinyl]-23,25-dimethoxy-13,19,21,27-tetramethyl-11,28-dioxa-4-azatricyclo[22.3.1.0^{4,9}]octacosa-14,18-diene-2,3,10,16-tetraone,
17-Allyl-1,2,14-trihydroxy-12-[2-(4-hydroxy-3-methocyclohexyl)-1-methylvinyl]-23,25-dimethoxy-13,19,21,27-tetramethyl-11,28-dioxa-4-azatricyclo[22.3.1.0^{4,9}]octacos-18-ene-3,10,16-trione,
17-Allyl-1,2,14,16-tetrahydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-3,10-dione,
17-Propyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacosane-2,3,10,16-tetraone,
17-Propyl-1,14-dihydroxy-12-[2-(4-hydroxy-3-methoxycyclohexyl)-1-methylethyl]-23,25-dimethoxy-13,19,21,27-tetramethyl-11,28-dioxa-4-azatricyclo[22.3.1.0^{4,9}]octacos-18-ene-2,3,10,16-tetraone,
17-Propyl-1,14-dihydroxy-12-[2-(4-hydroxy-3-methoxycyclohexyl)-1-methylethyl]-23,25-dimethoxy-13,19,21,27-tetramethyl-11,28-dioxa-4-azatricyclo[22.3.1.0^{4,9}]octacosane-2,3,10,16-tetraone,
17-Allyl-1,14,20-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone,
17-(1-Hydroxyprop-2-enyl)-1,14,20-trihydroxy-12-[2-(4-hydroxy-3-methoxycyclohexyl)-1-methylvinyl]-23,25-dimethoxy-13,19,21,27-tetramethyl-11,28-dioxa-4-azatricyclo [22.3.1.0^{4,9}]octacos-18-ene-2,3,10,16-tetraone,
17-Allyl-1,2-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-3,10,16-trione,
17-Allyl-1,16-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacosa-14,18-diene-2,3,10-trione,
17-Propyl-1-hydroxy-12-[2-(3-methoxy-4-oxocyclohexyl)-1-methylvinyl]-23,25-dimethoxy -13,19,21,27-tetramethyl-11,28-dioxa-4-azatricyclo[22.3.1.0^{4,9}]octacos-18-ene-2,3,10,16-tetraone,
17-(2,3-Dihydroxypropyl)-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}] octacos-18-ene-2,3,10,16-tetraone,
17-Ethanalyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone,
1,14-Dihydroxy-12-[2-(4-hydroxy-3-methoxycyclohexyl)-1-methylvinyl]-26,28-dimethoxy-13,22,24,30-tetramethyl-11,31-dioxa-4,17-diazatetracyclo[25.3.1.0^{4,9}.0^{16,20}]hentriaconta-16(20),18,21-triene-2,3,10-trione,
1,14-Dihydroxy-12-[2-(4-hydroxy-3-methoxycyclohexyl)-1-methylvinyl]-26,28-dimethoxy-17-(2-hydroxyethyl)-13,22,24,30-tetramethyl-11,31-dioxa-4,17-diazatetracyclo[25.3.1.0^{4,9}. 0^{16,20}]hentriaconta-16(20),18,21-triene-2,3,10-trione,
1,14-Dihydroxy-12-[2-(4-hydroxy-3-methoxycyclohexyl)-1-methylvinyl]-26,28-dimethoxy-13,22,24,30-tetramethyl-17-phenylmethyl-11,31-dioxa-4,17-diazatetracyclo [25.3.1.0^{4,9}.0^{16,20}]hentriaconta-16(20),18,21-triene-2,3,10-trione,
17-Allyl-1-hydroxy-12-[2-(3,4-epoxycyclohexyl)-1-methylvinyl]-23,25-dimethoxy-13,19,21,27-tetramethyl-11,28-dioxa-4-azatricyclo[22.3.1.0^{4,9}]octacosa-14,18-diene-2,3,10,16-tetraone,
17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone C16 oxime,
17-Allyl-1,14-dihydroxy-12-[2-(4-hydroxy-3-methoxycyclohexyl)-1-methvlvinyl]-23,25-dimethoxy-13,19,21,27-tetramethyl-11,28-dioxa-4-azatricyclo[22.3.1.0^{4,9}]octacos-18-ene-2,3,10,16-tetraone C16 oxime O-methyl ether,
17-Allyl-1,14-dihydroxy-12-[2-(4-(2',5'-dioxahexyloxy)-3-methoxycyclohexyl)-1-methylvinyl]-23,25-dimethoxy-13,19,21,27-tetramethyl-11,28-dioxa-4-azatricyclo [22.3.1.0^{4,9}]octacos-18-ene-2,3,10,16-tetraone,
17-Propyl-1-hydroxy-12-[2-(3,4-dihydroxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone,
1,14-Dihydroxy-12-[2-(4-hydroxy-3-methoxycyclohexyl)-1-methylvinyl]-23,25-dimethoxy-13,19,21,27-tetramethyl-17-(2-oxopropyl)-11,28-dioxa-4-azatricyclo[22.3.1.0^{4,9}]octacos-18-ene-2,3,10,16-tetraone,
17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-aza-spiro[tricyclo[22.3.1.0^{4,9}]octacos-18-ene-2,2'-oxirane]-3,10,16-trione,
17-Ethanalyl-1,2,14-trihydroxy-12-[2-(4-hydroxy-3-methoxycyclohexyl)-1-methvlvinyl]-23,25-dimethoxy-13,19,21,27-tetramethyl-11,28-dioxa-4-azatricyclo[22.3.1.0^{4,9}]octacos-18-ene-3,10,16-trione,
1,14-Dihydroxy-12-[2-(4-hydroxy-3-methoxycyclohexyl)-1-methylvinyl]-26,28-dimethoxy-18-[(phenylseleno)methyl]-13,22,24,30-tetramethyl-11,17,31-trioxa-4-azatetracyclo [25.3.1.0^{4,9}.0^{16,20}]hentriaconta-16(20),21-diene-2,3,10-trione,
Benzenesulphonic acid, 4'-methyl-[17-Allyl-1,14-dihydroxy-12-[2-(4-hydroxy-3-methoxycyclohexyl-1-methylvinyl]-23,25-dimethoxy-13,19,21,27-tetramethyl-11,28-dioxa -4-azatricyclo[22.3.1.0^{4,9}]octacos-18-ene-2,3,10-trione-16-ylidene]hydrazide,
or a pharmaceutically acceptable salt of any one thereof.

7. 17-Propyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone,
17-Allyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone,
or a pharmaceutically acceptable salt thereof.

8. The use of a compound of formula I, as defined in claim 1, or a compound according to claim 7, or a pharmaceutically acceptable salt thereof, as a pharmaceutical.

9. A pharmaceutical formulation comprising a compound of formula I as defined in claim 1, or a compound according to claim 7, or a pharmaceutically acceptable salt thereof, in admixture with a pharmaceutically acceptable adjuvant, diluent or carrier.

10. The use of a compound of formula I, as defined in claim 1, or a compound according to claim 7, or a pharmaceutically acceptable salt thereof, in the manufacture of a medicament for use as an immunosuppressive agent.

11. A process for the production of a compound of formula I, as defined in claim 1, or a compound according to claim 7, or a pharmaceutically acceptable salt thereof, which comprises:
(a) producing a compound of formula I, in which one or more of [R¹ and R²], [R³ and R⁴] or [R⁵ and R⁶] represent two vicinal hydrogen atoms, by selective reduction of a corresponding group [R¹ and R²], [R³ and R⁴] or [R⁵ and R⁶] when it represents a second bond between two vicinal carbon atoms in a corresponding compound,
(b) producing a compound of formula I, which contains one or more hydroxy groups, by selective reduction of one or more C=O groups in a corresponding compound,
(c) producing a compound of formula I, which contains a 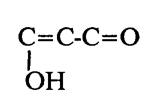 group, by selective oxidation of a 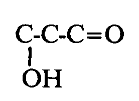 group
in a corresponding compound,
(d) producing a compound of formula I, which contains one or more alkoxy groups, by alkylation of one or more hydroxy groups in a corresponding compound by reaction with a suitable alkylating agent,
(e) producing a compound of formula I, which contains one or more hydroxyl groups, by deprotection of one or more protected hydroxy groups in a corresponding compound where the protecting group is preferably removable by hydrogenolysis,
(f) producing a compound of formula I, which contains a carbon-carbon double bond, by elimination of HL from a corresponding compound, where L is a leaving group,
(g) producing a compound of formula I, in which Y, R¹⁰ and R²³, together with the carbon atoms to which they are attached, form a tetrahydrofuran ring substituted by a CH₂Se(C₆H₅) group by reacting a phenylselenyl halide with a corresponding compound in which Y is O and R¹⁰ is allyl,
(h) producing a compound of formula I, which contains an allylic alcohol, by selective oxidation of an allyl group in a corresponding compound,
(i) producing a compound of formula I, which contains a ketone group, by oxidation of a hydroxy group in a corresponding compound,
(j) producing a compound of formula I, which contains a vicinal diol, by oxidation of a carbon-carbon double bond in a corresponding compound,
(k) producing a compound of formula I, which contains an aldehyde group, by oxidative cleavage of a vicinal diol in a corresponding compound,
(l) producing a compound of formula I, in which Y, R¹⁰ and R²³ together with the carbon atoms to which they are attached, form a pyrrole ring, by reacting ammonia or an amine with a corresponding compound in which Y is O and R¹⁰ is -CH₂CHO,
(m) producing a compound of formula I, which contains an epoxide group, by cyclization of an 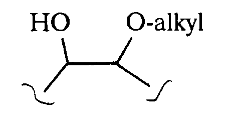 group in a corresponding compound,
(n) producing a compound of formula I, in which Y represents an oxime group, by reaction of a corresponding compound in which Y is O with an oxygen-substituted amine,
(o) producing a compound of formula I, which contains a COCH₃ group, by oxidation of a terminal alkene group in a corresponding compound,
(p) producing a compound of formula I, in which X represents -CH₂O-, by reacting a corresponding compound in which X is O with diazomethane, or
(q) producing a compound of formula I, in which Y is a hydrazone or a hydrazone derivative, by reacting a corresponding compound in which Y is O with hydrazine or a substituted hydrazine.

## Claims (Claims for the following Contracting State(s): AT, BE, LU, SE)

1. A compound of formula I, 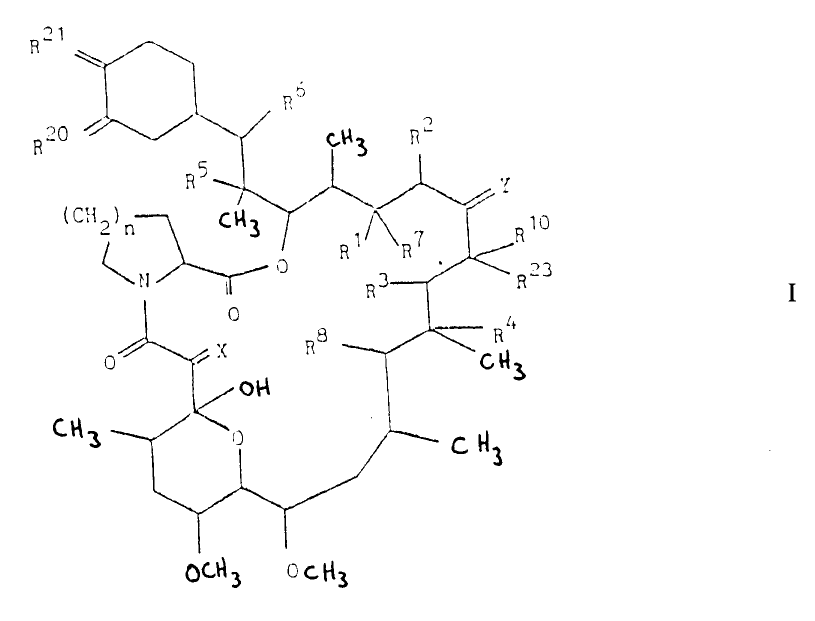 wherein each vicinal pair of substituents [R¹ and R²], [R³ and R⁴] and [R⁵ and R⁶] independently:
a) represent two vicinal hydrogen atoms, or
b) form a second bond between the vicinal carbon atoms to which they are attached;
R⁷ represents H, OH or O-alkyl C₁₋₆, or in conjunction with R¹ it may represent =O;
R⁸ represents H or OH;
R¹⁰ represents alkyl C₁₋₆, alkyl C₁₋₆ substituted by one or more hydroxy groups, alkenyl C₂₋₆, alkenyl C₂₋₆ substituted by one or more hydroxy groups, or alkyl C₁₋₆ substituted by =O;
X represents O, (H,OH) or -CH₂O-;
Y represents O, (H,OH), N-NR¹¹R¹² or N-OR¹³;
R¹¹ and R¹² independently represent H, alkyl C₁₋₆, phenyl or tosyl;
R¹³ represents H or alkyl C₁₋₆;
R²⁰ and R²¹ independently represent O, or they may independently represent (R²⁰a,H) and (R²¹a,H) respectively; R²⁰a and R²¹a independently represent OH, O-alkyl C₁₋₆ or OCH₂OCH₂CH₂OCH₃; in addition R²⁰a and R²¹a may together represent an oxygen atom in an epoxide ring;
R²³ represents H;
n is 1 or 2;
in addition to their significances above, Y, R¹⁰ and R²³, together with the carbon atoms to which they are attached, may represent a 5- or 6-membered N-, S- or O-containing heterocyclic ring, which may be saturated or unsaturated, and which may be substituted by one or more groups selected from alkyl C₁₋₆, hydroxy, alkyl C₁₋₆ substituted by one or more hydroxy groups, O-alkyl C₁₋₆, benzyl and -CH₂Se(C₆H₅);
provided that:
(a) when [R³ and R⁴] and [R⁵ and R⁶] each represent a carbon-carbon bond; R⁷ represents H or an OH group; R⁸ represents H; R¹⁰ represents methyl, ethyl, propyl or allyl; R²⁰a represents OCH₃; and R²¹a represents OH; then X and Y do not both represent O; and
(b) when [R¹ and R²] represents two vicinal hydrogen atoms; [R³ and R⁴] and [R⁵ and R⁶] each represent a carbon-carbon bond; R⁷ represents an OH group; R⁸ represents H; R¹⁰ represents allyl; R²¹a represents OH; X and Y each represent O; and n is 2; then R²⁰ does not represent O or (OH,H);
and pharmaceutically acceptable salts thereof.

2. A compound according to claim 1, wherein R¹⁰ represents methyl, ethyl, propyl or allyl.

3. A compound according to claim 1 or claim 2, wherein at least one of R²⁰a and R²¹a represents OH or OCH₃.

4. A compound according to any one of the preceding claims, wherein n is 2.

5. A compound according to any one of the preceding claims, wherein R⁷ represents H or OH.

6. A compound according to claim 1, which is:
17-Allyl-1-hydroxy-12-[2-(3,4-dimethoxycyclohexyl)-1-methylvinyl]-14,23,25-trimethoxy-13,19,21,27-tetramethyl-11,28-dioxa-4-azatricyclo[22.3.1.0^{4,9}]octacos-18-ene-2,3,10,16-tetraone,
17-Allyl-1,14-dihydroxy-12-[2-(3,4-dimethoxycyclohexyl)-1-methylvinyl]-23,25-dimethoxy -13,19,21,27-tetramethyl-11,28-dioxa-4-azatricyclo[22.3.1.0^{4,9}]octacos-18-ene-2,3,10,16-tetraone,
1,14-Dihydroxy-12-[2-(4-hydroxy-3-methoxycyclohexyl)-1-methylvinyl]-18-[(phenylseleno)methyl]-16,26,28-trimethoxy-13,22,24,30-tetramethyl-11,17,31-trioxa-4-azatetracyclo[25.3.1.0^{4,9}.0^{16,20}]hentriacont-21-ene-2,3,10-trione,
17-Allyl-1-hydroxy-12-[2-(3,4-dimethoxycyclohexyl)-1-methylvinyl]-23,25-dimethoxy-13,19,21,27-tetramethyl-11,28-dioxa-4-azatricyclo[22.3.1.0^{4,9}]octacosa-14,18-diene-2,3,10,16-tetraone,
17-Allyl-1,2,14-trihydroxy-12-[2-(4-hydroxy-3-methocyclohexyl)-1-methylvinyl]-23,25-dimethoxy-13,19,21,27-tetramethyl-11,28-dioxa-4-azatricyclo[22.3.1.0^{4,9}]octacos-18-ene-3,10,16-trione,
17-Allyl-1,2,14,16-tetrahydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-3,10-dione,
17-Propyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacosane-2,3,10,16-tetraone,
17-Propyl-1,14-dihydroxy-12-[2-(4-hydroxy-3-methoxycyclohexyl)-1-methylethyl]-23,25-dimethoxy-13,19,21,27-tetramethyl-11,28-dioxa-4-azatricyclo[22.3.1.0^{4,9}]octacos-18-ene-2,3,10,16-tetraone,
17-Propyl-1,14-dihydroxy-12-[2-(4-hydroxy-3-methoxycyclohexyl)-1-methylethyl]-23,25-dimethoxy-13,19,21,27-tetramethyl-11,28-dioxa-4-azatricyclo[22.3.1.0^{4,9}]octacosane-2,3,10,16-tetraone,
17-Allyl-1,14,20-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone,
17-(1-Hydroxyprop-2-enyl)-1,14,20-trihydroxy-12-[2-(4-hydroxy-3-methoxycyclohexyl)-1-methylvinyl]-23,25-dimethoxy-13,19,21,27-tetramethyl-11,28-dioxa-4-azatricyclo [22.3.1.0^{4,9}]octacos-18-ene-2,3,10,16-tetraone,
17-Allyl-1,2-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-3,10,16-trione,
17-Allyl-1,16-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacosa-14,18-diene-2,3,10-trione,
17-Propyl-1-hydroxy-12-[2-(3-methoxy-4-oxocyclohexyl)-1-methylvinyl]-23,25-dimethoxy -13,19,21,27-tetramethyl-11,28-dioxa-4-azatricyclo[22.3.1.0^{4,9}]octacos-18-ene-2,3,10,16-tetraone,
17-(2,3-Dihydroxypropyl)-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}] octacos-18-ene-2,3,10,16-tetraone,
17-Ethanalyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone,
1,14-Dihydroxy-12-[2-(4-hydroxy-3-methoxycyclohexyl)-1-methylvinyl]-26,28-dimethoxy-13,22,24,30-tetramethyl-11,31-dioxa-4,17-diazatetracyclo[25.3.1.0^{4,9}.0^{16,20}]hentriaconta-16(20),18,21-triene-2,3,10-trione,
1,14-Dihydroxy-12-[2-(4-hydroxy-3-methoxycyclohexyl)-1-methylvinyl]-26,28-dimethoxy-17-(2-hydroxyethyl)-13,22,24,30-tetramethyl-11,31-dioxa-4,17-diazatetracyclo[25.3.1.0^{4,9}. 0^{16,20}]hentriaconta-16(20),18,21-triene-2,3,10-trione,
1,14-Dihydroxy-12-[2-(4-hydroxy-3-methoxycyclohexyl)-1-methylvinyl]-26,28-dimethoxy-13,22,24,30-tetramethyl-17-phenylmethyl-11,31-dioxa-4,17-diazatetracyclo [25.3.1.0^{4,9}.0^{16,20}]hentriaconta-16(20),18,21-triene-2,3,10-trione,
17-Allyl-1-hydroxy-12-[2-(3,4-epoxycyclohexyl)-1-methylvinyl]-23,25-dimethoxy-13,19,21,27-tetramethyl-11,28-dioxa-4-azatricyclo[22.3.1.0^{4,9}]octacosa-14,18-diene-2,3,10,16-tetraone,
17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone C16 oxime,
17-Allyl-1,14-dihydroxy-12-[2-(4-hydroxy-3-methoxycyclohexyl)-1-methvlvinyl]-23,25-dimethoxy-13,19,21,27-tetramethyl-11,28-dioxa-4-azatricyclo[22.3.1.0^{4,9}]octacos-18-ene-2,3,10,16-tetraone C16 oxime O-methyl ether,
17-Allyl-1,14-dihydroxy-12-[2-(4-(2',5'-dioxahexyloxy)-3-methoxycyclohexyl)-1-methylvinyl]-23,25-dimethoxy-13,19,21,27-tetramethyl-11,28-dioxa-4-azatricyclo [22.3.1.0^{4,9}]octacos-18-ene-2,3,10,16-tetraone,
17-Propyl-1-hydroxy-12-[2-(3,4-dihydroxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone,
1,14-Dihydroxy-12-[2-(4-hydroxy-3-methoxycyclohexyl)-1-methylvinyl]-23,25-dimethoxy-13,19,21,27-tetramethyl-17-(2-oxopropyl)-11,28-dioxa-4-azatricyclo[22.3.1.0^{4,9}]octacos-18-ene-2,3,10,16-tetraone,
17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-aza-spiro[tricyclo[22.3.1.0^{4,9}]octacos-18-ene-2,2'-oxirane]-3,10,16-trione,
17-Ethanalyl-1,2,14-trihydroxy-12-[2-(4-hydroxy-3-methoxycyclohexyl)-1-methvlvinyl]-23,25-dimethoxy-13,19,21,27-tetramethyl-11,28-dioxa-4-azatricyclo[22.3.1.0^{4,9}]octacos-18-ene-3,10,16-trione,
1,14-Dihydroxy-12-[2-(4-hydroxy-3-methoxycyclohexyl)-1-methylvinyl]-26,28-dimethoxy-18-[(phenylseleno)methyl]-13,22,24,30-tetramethyl-11,17,31-trioxa-4-azatetracyclo [25.3.1.0^{4,9}.0^{16,20}]hentriaconta-16(20),21-diene-2,3,10-trione,
Benzenesulphonic acid, 4'-methyl-[17-Allyl-1,14-dihydroxy-12-[2-(4-hydroxy-3-methoxycyclohexyl-1-methylvinyl]-23,25-dimethoxy-13,19,21,27-tetramethyl-11,28-dioxa -4-azatricyclo[22.3.1.0^{4,9}]octacos-18-ene-2,3,10-trione-16-ylidene]hydrazide,
or a pharmaceutically acceptable salt of any one thereof.

7. 17-Propyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone,
17-Allyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone,
or a pharmaceutically acceptable salt thereof.

8. The use of a compound of formula I, as defined in claim 1, or a compound according to claim 7, or a pharmaceutically acceptable salt thereof, as a pharmaceutical.

9. A pharmaceutical formulation comprising a compound of formula I as defined in claim 1, or a compound according to claim 7, or a pharmaceutically acceptable salt thereof, in admixture with a pharmaceutically acceptable adjuvant, diluent or carrier.

10. The use of a compound of formula I, as defined in claim 1, or a compound according to claim 7, or a pharmaceutically acceptable salt thereof, in the manufacture of a medicament for use as an immunosuppressive agent.

11. A process for the production of a compound of formula I, as defined in claim 1, or a compound according to claim 7, or a pharmaceutically acceptable salt thereof, which comprises:
(a) producing a compound of formula I, in which one or more of [R¹ and R²], [R³ and R⁴] or [R⁵ and R⁶] represent two vicinal hydrogen atoms, by selective reduction of a corresponding group [R¹ and R²], [R³ and R⁴] or [R⁵ and R⁶] when it represents a second bond between two vicinal carbon atoms in a corresponding compound,
(b) producing a compound of formula I, which contains one or more hydroxy groups, by selective reduction of one or more C=O groups in a corresponding compound,
(c) producing a compound of formula I, which contains a 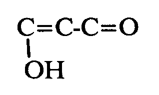 group, by selective oxidation of a 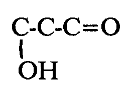 group
in a corresponding compound,
(d) producing a compound of formula I, which contains one or more alkoxy groups, by alkylation of one or more hydroxy groups in a corresponding compound by reaction with a suitable alkylating agent,
(e) producing a compound of formula I, which contains one or more hydroxyl groups, by deprotection of one or more protected hydroxy groups in a corresponding compound where the protecting group is preferably removable by hydrogenolysis,
(f) producing a compound of formula I, which contains a carbon-carbon double bond, by elimination of HL from a corresponding compound, where L is a leaving group,
(g) producing a compound of formula I, in which Y, R¹⁰ and R²³, together with the carbon atoms to which they are attached, form a tetrahydrofuran ring substituted by a CH₂Se(C₆H₅) group by reacting a phenylselenyl halide with a corresponding compound in which Y is O and R¹⁰ is allyl,
(h) producing a compound of formula I, which contains an allylic alcohol, by selective oxidation of an allyl group in a corresponding compound,
(i) producing a compound of formula I, which contains a ketone group, by oxidation of a hydroxy group in a corresponding compound,
(j) producing a compound of formula I, which contains a vicinal diol, by oxidation of a carbon-carbon double bond in a corresponding compound,
(k) producing a compound of formula I, which contains an aldehyde group, by oxidative cleavage of a vicinal diol in a corresponding compound,
(l) producing a compound of formula I, in which Y, R¹⁰ and R²³ together with the carbon atoms to which they are attached, form a pyrrole ring, by reacting ammonia or an amine with a corresponding compound in which Y is O and R¹⁰ is -CH₂CHO,
(m) producing a compound of formula I, which contains an epoxide group, by cyclization of an 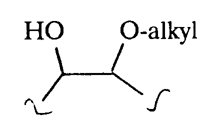 group in a corresponding compound,
(n) producing a compound of formula I, in which Y represents an oxime group, by reaction of a corresponding compound in which Y is O with an oxygen-substituted amine,
(o) producing a compound of formula I, which contains a COCH₃ group, by oxidation of a terminal alkene group in a corresponding compound,
(p) producing a compound of formula I, in which X represents -CH₂O-, by reacting a corresponding compound in which X is O with diazomethane, or
(q) producing a compound of formula I, in which Y is a hydrazone or a hydrazone derivative, by reacting a corresponding compound in which Y is O with hydrazine or a substituted hydrazine.

## Claims (Claims for the following Contracting State(s): ES, GR)

1. A process for the production of a compound of formula I, 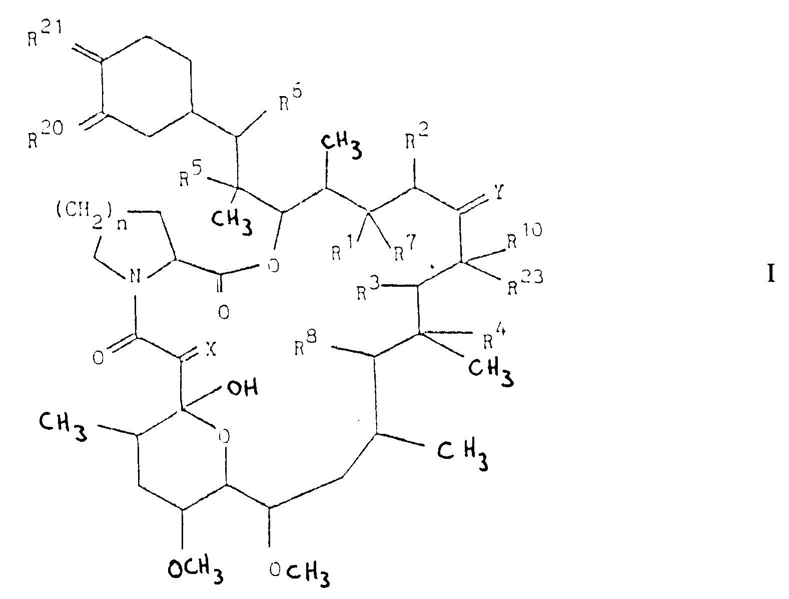 wherein each vicinal pair of substituents [R¹ and R²], [R³ and R⁴] and [R⁵ and R⁶] independently:
a) represent two vicinal hydrogen atoms, or
b) form a second bond between the vicinal carbon atoms to which they are attached;
R⁷represents H, OH or O-alkyl C₁₋₆, or in conjunction with R¹ it may represent =O;
R⁸ represents H or OH;
R¹⁰ represents alkyl C₁₋₆, alkyl C₁₋₆ substituted by one or more hydroxy groups, alkenyl C₂₋₆, alkenyl C₂₋₆ substituted by one or more hydroxy groups, or alkyl C₁₋₆ substituted by =O;
X represents O, (H,OH) or -CH₂O-;
Y represents O, (H,OH), N-NR¹¹R¹² or N-OR¹³;
R¹¹ and R¹² independently represent H, alkyl C₁₋₆, phenyl or tosyl;
R¹³ represents H or alkyl C₁₋₆;
R²⁰ and R²¹ independently represent O, or they may independently represent (R²⁰a,H) and (R²¹a,H) respectively; R²⁰a and R²¹a independently represent OH, O-alkyl C₁₋₆ or OCH₂OCH₂CH₂OCH₃; in addition R²⁰a and R²¹a may together represent an oxygen atom in an epoxide ring;
R²³ represents H;
n is 1 or 2;
in addition to their significances above, Y, R¹⁰ and R²³, together with the carbon atoms to which they are attached, may represent a 5- or 6-membered N-, S- or O-containing heterocyclic ring, which may be saturated or unsaturated, and which may be substituted by one or more groups selected from alkyl C₁₋₆, hydroxy, alkyl C₁₋₆ substituted by one or more hydroxy groups, O-alkyl C₁₋₆, benzyl and -CH₂Se(C₆H₅);
provided that:
(a) when [R³ and R⁴] and [R⁵ and R⁶] each represent a carbon-carbon bond; R⁷ represents H or an OH group; R⁸ represents H; R¹⁰ represents methyl, ethyl, propyl or allyl; R²⁰a represents OCH₃; and R²¹a represents OH; then X and Y do not both represent O; and
(b) when [R¹ and R²] represents two vicinal hydrogen atoms; [R³ and R⁴] and [R⁵ and R⁶] each represent a carbon-carbon bond; R⁷ represents an OH group; R⁸ represents H; R¹⁰ represents allyl; R²¹a represents OH; X and Y each represent O; and n is 2; then R²⁰ does not represent O or (OH,H);
and pharmaceutically acceptable salts thereof, which comprises:
(a) producing a compound of formula I, in which one or more of [R¹ and R²], [R³ and R⁴] or [R⁵ and R⁶] represent two vicinal hydrogen atoms, by selective reduction of a corresponding group [R¹ and R²], [R³ and R⁴] or [R⁵ and R⁶] when it represents a second bond between two vicinal carbon atoms in a corresponding compound,
(b) producing a compound of formula I, which contains one or more hydroxy groups, by selective reduction of one or more C=O groups in a corresponding compound,
(c) producing a compound of formula I, which contains a 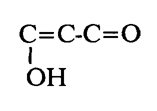 group, by selective oxidation of a 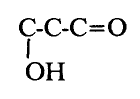 group
in a corresponding compound,
(d) producing a compound of formula I, which contains one or more alkoxy groups, by alkylation of one or more hydroxy groups in a corresponding compound by reaction with a suitable alkylating agent,
(e) producing a compound of formula I, which contains one or more hydroxyl groups, by deprotection of one or more protected hydroxy groups in a corresponding compound where the protecting group is preferably removable by hydrogenolysis,
(f) producing a compound of formula I, which contains a carbon-carbon double bond, by elimination of HL from a corresponding compound, where L is a leaving group,
(g) producing a compound of formula I, in which Y, R¹⁰ and R²³, together with the carbon atoms to which they are attached, form a tetrahydrofuran ring substituted by a CH₂Se(C₆H₅) group by reacting a phenylselenyl halide with a corresponding compound in which Y is O and R¹⁰ is allyl,
(h) producing a compound of formula I, which contains an allylic alcohol, by selective oxidation of an allyl group in a corresponding compound,
(i) producing a compound of formula I, which contains a ketone group, by oxidation of a hydroxy group in a corresponding compound,
(j) producing a compound of formula I, which contains a vicinal diol, by oxidation of a carbon-carbon double bond in a corresponding compound,
(k) producing a compound of formula I, which contains an aldehyde group, by oxidative cleavage of a vicinal diol in a corresponding compound,
(l) producing a compound of formula I, in which Y, R¹⁰ and R²³ together with the carbon atoms to which they are attached, form a pyrrole ring, by reacting ammonia or an amine with a corresponding compound in which Y is O and R¹⁰ is -CH₂CHO,
(m) producing a compound of formula I, which contains an epoxide group, by cyclization of an 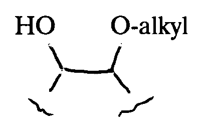 group in a corresponding compound,
(n) producing a compound of formula I, in which Y represents an oxime group, by reaction of a corresponding compound in which Y is O with an oxygen-substituted amine,
(o) producing a compound of formula I, which contains a COCH₃ group, by oxidation of a terminal alkene group in a corresponding compound,
(p) producing a compound of formula I, in which X represents -CH₂O-, by reacting a corresponding compound in which X is O with diazomethane, or
(q) producing a compound of formula I, in which Y is a hydrazone or a hydrazone derivative, by reacting a corresponding compound in which Y is O with hydrazine or a substituted hydrazine.

2. A process according to claim 1, wherein R¹⁰ represents methyl, ethyl, propyl or allyl.

3. A process according to claim 1 or claim 2, wherein at least one of R²⁰a and R²¹a represents OH or OCH₃.

4. A process according to any one of the preceding claims, wherein n is 2.

5. A process according to any one of the preceding claims, wherein R⁷ represents H or OH.

6. A process according to claim 1, wherein the compound of formula I is:
17-Allyl-1-hydroxy-12-[2-(3,4-dimethoxycyclohexy)-1-methylvinyl]-14,23,25-trimethoxy-13,19,21,27-tetramethyl-11,28-dioxa-4-azatricyclo[22.3.1.0^{4,9}]octacos-18-ene-2,3,10,16-tetraone,
17-Allyl-1,14-dihydroxy-12-[2-(3,4-dimethoxycyclohexyl)-1-methylvinyl]-23,25-dimethoxy -13,19,21,27-tetramethyl-11,28-dioxa-4-azatricyclo[22.3.1.0^{4,9}]octacos-18-ene-2,3,10,16-tetraone,
1,14-Dihydroxy-12-[2-(4-hydroxy-3-methoxycyclohexyl)-1-methylvinyl]-18-[(phenylseleno)methyl]-16,26,28-trimethoxy-13,22,24,30-tetramethyl-11,17,31-trioxa-4-azatetracyclo[25.3.1.0^{4,9}.0^{16,20}]hentriacont-21-ene-2,3,10-trione,
17-Allyl-1-hydroxy-12-[2-(3,4-dimethoxycyclohexy)-1-methylvinyl]-23,25-dimethoxy-13,19,21,27-tetramethyl-11,28-dioxa-4-azatricyclo[22.3.1.0^{4,9}]octacosa-14,18-diene-2,3,10,16-tetraone,
17-Allyl-1,2,14-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-3,10,16-trione,
17-Allyl-1,2,14,16-tetrahydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-3,10-dione,
17-Propyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacosane-2,3,10,16-tetraone,
17-Propyl-1,14-dihydroxy-12-[2-(4-hydroxy-3-methoxycyclohexyl)-1-methylethyl]-23,25-dimethoxy-13,19,21,27-tetramethyl-11,28-dioxa-4-azatricyclo[22.3.1.0^{4,9}]octacos-18-ene-2,3,10,16-tetraone,
17-Propyl-1,14-dihydroxy-12-[2-(4-hydroxy-3-methoxycyclohexyl)-1-methylethyl]-23,25-dimethoxy-13,19,21,27-tetramethyl-11,28-dioxa-4-azatricyclo[22.3.1.0^{4,9}]octacosane-2,3,10,16-tetraone,
17-Allyl-1,14,20-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone,
17-(1-Hydroxyprop-2-enyl)-1,14,20-trihydroxy-12-[2-(4-hydroxy-3-methoxycyclohexyl)-1-methylvinyl]-23,25-dimethoxy-13,19,21,27-tetramethyl-11,28-dioxa-4-azatricyclo [22.3.1.0^{4,9}]octacos-18-ene-2,3,10,16-tetraone,
17-Allyl-1,2-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-3,10,16-trione,
17-Allyl-1,16-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacosa-14,18-diene-2,3,10-trione,
17-Propyl-1-hydroxy-12-[2-(3-methoxy-4-oxocyclohexyl)-1-methylvinyl]-23,25-dimethoxy -13,19,21,27-tetramethyl-11,28-dioxa-4-azatricyclo[22.3.1.0^{4,9}]octacos-18-ene-2,3,10,16-tetraone,
17-(2,3-Dihydroxypropyl)-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}] octacos-18-ene-2,3,10,16-tetraone,
17-Ethanalyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone,
1,14-Dihydroxy-12-[2-(4-hydroxy-3-methoxycyclohexyl)-1-methylvinyl]-26,28-dimethoxy-13,22,24,30-tetramethyl-11,31-dioxa-4,17-diazatetracyclo[25.3.1.0^{4,9}.0^{16,20}]hentriaconta-16(20),18,21-triene-2,3,10-trione,
1,14-Dihydroxy-12-[2-(4-hydroxy-3-methoxycyclohexyl)-1-methylvinyl]-26,28-dimethoxy-17-(2-hydroxyethyl)-13,22,24,30-tetramethyl-11,31-dioxa-4,17-diazatetracyclo[25.3.1.0^{4,9}. 0^{16,20}]hentriaconta-16(20),18,21-triene-2,3,10-trione,
1,14-Dihydroxy-12-[2-(4-hydroxy-3-methoxycyclohexyl)-1-methylvinyl]-26,28-dimethoxy-13,22,24,30-tetramethyl-17-phenylmethyl-11,31-dioxa-4,17-diazatetracyclo [25.3.1.0^{4,9}.0^{16,20}]hentriaconta-16(20),18,21-triene-2,3,10-trione,
17-Allyl-1-hydroxy-12-[2-(3,4-epoxycyclohexyl)-1-methylvinyl]-23,25-dimethoxy-13,19,21,27-tetramethyl-11,28-dioxa-4-azatricyclo[22.3.1.0^{4,9}]octacosa-14,18-diene-2,3,10,16-tetraone,
17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone C16 oxime,
17-Allyl-1,14-dihydroxy-12-[2-(4-hydroxy-3-methoxycyclohexyl)-1-methvlvinyl]-23,25-dimethoxy-13,19,21,27-tetramethyl-11,28-dioxa-4-azatricyclo[22.3.1.0^{4,9}]octacos-18-ene-2,3,10,16-tetraone C16 oxime O-methyl ether,
17-Allyl-1,14-dihydroxy-12-[2-(4-(2',5'-dioxahexyloxy)-3-methoxycyclohexyl)-1-methylvinyl]-23,25-dimethoxy-13,19,21,27-tetramethyl-11,28-dioxa-4-azatricyclo [22.3.1.0^{4,9}]octacos-18-ene-2,3,10,16-tetraone,
17-Propyl-1-hydroxy-12-[2-(3,4-dihydroxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone,
1,14-Dihydroxy-12-[2-(4-hydroxy-3-methoxycyclohexyl)-1-methylvinyl]-23,25-dimethoxy-13,19,21,27-tetramethyl-17-(2-oxopropyl)-11,28-dioxa-4-azatricyclo[22.3.1.0^{4,9}]octacos-18-ene-2,3,10,16-tetraone,
17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-aza-spiro[tricyclo[22.3.1.0^{4,9}]octacos-18-ene-2,2'-oxirane]-3,10,16-trione,
17-Ethanalyl-1,2,14-trihydroxy-12-[2-(4-hydroxy-3-methoxycyclohexyl)-1-methvlvinyl]-23,25-dimethoxy-13,19,21,27-tetramethyl-11,28-dioxa-4-azatricyclo[22.3.1.0^{4,9}]octacos-18-ene-3,10,16-trione,
1,14-Dihydroxy-12-[2-(4-hydroxy-3-methoxycyclohexyl)-1-methylvinyl]-26,28-dimethoxy-18-[(phenylseleno)methyl]-13,22,24,30-tetramethyl-11,17,31-trioxa-4-azatetracyclo [25.3.1.0^{4,9}.0^{16,20}]hentriaconta-16(20),21-diene-2,3,10-trione,
Benzenesulphonic acid, 4'-methyl-[17-Allyl-1,14-dihydroxy-12-[2-(4-hydroxy-3-methoxycyclohexyl-1-methylvinyl]-23,25-dimethoxy-13,19,21,27-tetramethyl-11,28-dioxa -4-azatricyclo[22.3.1.0^{4,9}]octacos-18-ene-2,3,10-trione-16-ylidene]hydrazide,
or a pharmaceutically acceptable salt of any one thereof.

7. A process for the production of:
17-Propyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone,
17-Allyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-ene-2,3,10,16-tetraone,
or a pharmaceutically acceptable salt thereof,
which comprises selective reduction of a corresponding compound having a carbon-carbon double bond in the 14-position.

8. A process for the production of a pharmaceutical formulation comprising as active ingredient a compound of formula I as defined in claim 1, or a compound as defined in claim 7, or a pharmaceutically acceptable salt thereof, which comprises mixing the active ingredient with a pharmaceutically acceptable adjuvant, diluent or carrier.

## Patentansprüche (Patentansprüche für folgende(n) Vertragsstaat(en): CH, LI, DE, FR, GB, IT, NL)

1. Verbindung der Formel (I) 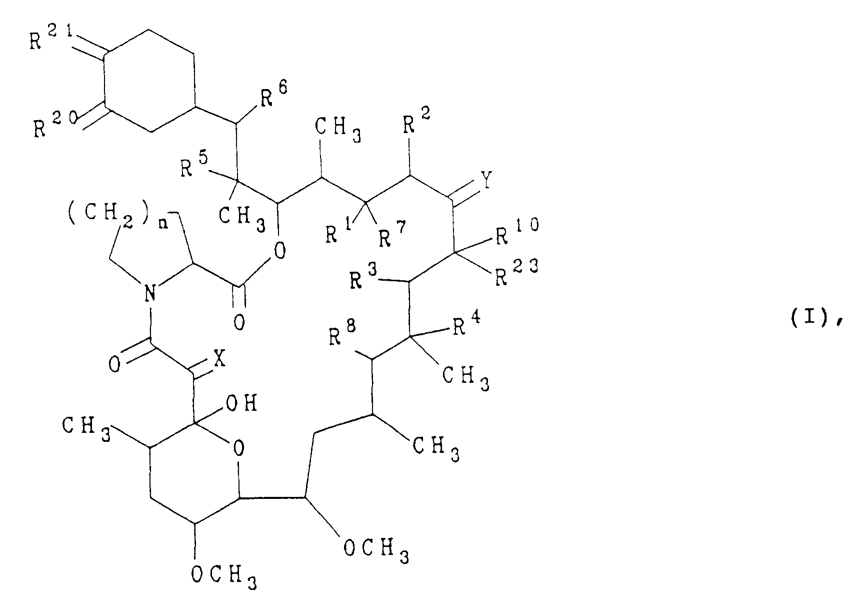 worin jedes benachbarte Substituentenpaar [R¹ und R²],
[R³ und R⁴] und [R⁵ und R⁶] unabhängig:
a) zwei benachbarte Wasserstoffatome darstellt, oder
b) eine zweite Bindung zwischen den benachbarten Kohlenstoffatomen, an die sie gebunden sind, bildet;
R⁷ die Bedeutung H, OH oder O-C₁-C₆-Alkyl hat, oder zusammen mit R¹ =O darstellen kann;
R⁸ die Bedeutung H oder OH hat;
R¹⁰ C₁-C₆-Alkyl, C₁-C₆-Alkyl substituiert durch eine oder mehrere Hydroxy-Gruppen, C₂-C₆-Alkenyl, C₂-C₆-Alkenyl substituiert durch eine oder mehrere Hydroxy-Gruppen, oder C₁-C₆-Alkyl substituiert durch =O ist;
X die Bedeutung O, (H,OH) oder -CH₂O- hat;
Y die Bedeutung O, (H,OH), N-NR¹¹R¹² oder N-OR¹³ hat;
R¹¹ und R¹² unabhängig H, C₁-C₆-Alkyl, Phenyl oder Tosyl sind;
R¹³ die Bedeutung H oder C₁-C₆-Alkyl hat;
R²⁰ und R²¹ unabhängig O darstellen, oder unabhängig (R²⁰a,H) bzw. (R²¹a,H) sein können; R²⁰a und R²¹a unabhängig OH, O-C₁-C₆-Alkyl oder OCH₂OCH₂CH₂OCH₃ darstellen; außerdem R²⁰a und R²¹a gemeinsam ein Sauerstoffatom in einem Epoxidring bedeuten können;
R²³ H darstellt;
n 1 oder 2 ist;
zusätzlich zu ihren obigen Bedeutungen Y, R¹⁰ und R²³ gemeinsam mit den Kohlenstoffatomen, an die sie gebunden sind, darstellen können: einen 5- oder 6-gliedrigen, N-, S- oder O-haltigen heterocyclischen Ring, der gesättigt oder ungesättigt sein kann, und der substituiert sein kann durch eine oder mehrere Gruppen, ausgewählt aus C₁-C₆-Alkyl, Hydroxy, C₁-C₆-Alkyl substituiert durch eine oder mehrere Hydroxy-Gruppen, O-C₁-C₆-Alkyl, Benzyl und -CH₂Se(C₆H₅);
vorausgesetzt, daß:
(a) wenn [R³ und R⁴] und [R⁵ und R⁶] jeweils eine Kohlenstoff-Kohlenstoff-Bindung darstellen; R⁷ H oder eine OH Gruppe bedeutet; R⁸ H ist; R¹⁰ Methyl, Ethyl, Propyl oder Allyl bedeutet; R²⁰a OCH₃ darstellt; und R²¹a OH ist; X und Y nicht beide O sind;
(b) wenn [R¹ und R²] zwei benachbarte Wasserstoffatome darstellt; [R³ und R⁴] und [R⁵ und R⁶] jeweils eine Kohlenstoff-Kohlenstoff-Bindung sind; R⁷ eine OH Gruppe bedeutet; R⁸ H ist; R¹⁰ Allyl bedeutet; R²¹a OH ist; X und Y beide O sind; und n 2 ist; R²⁰ nicht O oder (OH,H) darstellt; und
(c) wenn [R¹ und R²] zwei benachbarte Wasserstoffatome darstellt; [R³ und R⁴] und [R⁵ und R⁶] jeweils eine Kohlenstoff-Kohlenstoff-Bindung sind; R⁷ eine OH Gruppe bedeutet; R⁸ H ist; R¹⁰ Ethyl bedeutet; R²¹a OH ist; X und Y jeweils O sind; und n 2 ist; R²⁰a nicht OH darstellt;
und pharmazeutisch annehmbare Salze hievon.

2. Verbindung nach Anspruch 1, worin R¹⁰ Methyl, Ethyl, Propyl oder Allyl bedeutet.

3. Verbindung nach Anspruch 1 oder 2, worin zumindest eines von R²⁰a und R²¹a die Bedeutung OH oder OCH₃ hat.

4. Verbindung nach einem der vorhergehenden Ansprüche, worin n 2 ist.

5. Verbindung nach einem der vorhergehenden Ansprüche, worin R⁷ H oder OH darstellt.

6. Verbindung nach Anspruch 1, nämlich:
17-Allyl-1-hydroxy-12-[2-(3,4-dimethoxycyclohexyl)-1-methylvinyl]-14,23,25-trimethoxy-13,19,21,27-tetramethyl-11,28-dioxa-4-azatricyclo[22.3.1.0^{4,9}]octacos-18-en-2,3,10,16-tetraon,
17-Allyl-1,14-dihydroxy-12-[2-(3,4-dimethoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
1,14-Dihydroxy-12-[2-(4-hydroxy-3-methoxycyclohexyl)-1-methylvinyl]-18-[(phenylseleno)-methyl]-16,26,28-trimethoxy-13, 22,24,30-tetramethyl-11,17,31-trioxa-4-azatetracyclo-[25.3.1.0^{4,9}.0^{16,20}]hentriacont-21-en-2,3,10-trion,
17-Allyl-1-hydroxy-12-[2-(3,4-dimethoxycyclohexyl)-1-methylvinyl]-23,25-dimethoxy-13,19,21,27-tetramethyl-11,28-dioxa-4-azatricyclo[22.3.1.0^{4,9}]octacosa-14,18-dien-2,3,10,16-tetraon,
17-Allyl-1,2,14-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-3,10,16-trion,
17-Allyl-1,2,14,16-tetrahydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-3,10-dion,
17-Propyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacosan-2,3,10,16-tetraon,
17-Propyl-1,14-dihydroxy-12-[2-(4-hydroxy-3-methoxycyclohexyl)-1-methylethyl]-23,25-dimethoxy-13,19,21,27-tetramethyl-11,28-dioxa-4-azatricyclo[22.3.1.0^{4,9}]octacos-18-en-2,3,10,16-tetraon,
17-Propyl-1,14-dihydroxy-12-[2-(4-hydroxy-3-methoxycyclohexyl)-1-methylethyl]-23,25-dimethoxy-13,19,21,27-tetramethyl-11,28-dioxa-4-azatricyclo[22.3.1.0^{4,9}]octacosan-2,3,10,16-tetraon,
17-Allyl-1,14,20-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-(1-Hydroxyprop-2-enyl)-1,14,20-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-Allyl-1,2-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-3,10,16-trion,
17-Allyl-1,16-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacosa-14,18-dien-2,3,10-trion,
17-Propyl-1-hydroxy-12-[2-(3-methoxy-4-oxocyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-(2,3-Dihydroxypropyl)-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-Ethanalyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
1,14-Dihydroxy-12-[2-(4-hydroxy-3-methoxycyclohexyl)-1-methylvinyl]-26,28-dimethoxy-13,22,24,30-tetramethyl-11,31-dioxa-4,17-diazatetracyclo[25.3.1.0^{4,9}.0^{16,20}]hentriaconta-16(20),18,21-trien-2,3,10-trion,
1,14-Dihydroxy-12-[2-(4-hydroxy-3-methoxycyclohexyl)-1-methylvinyl]-26,28-dimethoxy-17-(2-hydroxyethyl)-13,22,24,30-tetramethyl-11,31-dioxa-4,17-diazatetracyclo[25.3.1.0^{4,9}.0^{16,20}]-hentriaconta-16(20),18,21-trien-2,3,10-trion,
1,14-Dihydroxy-12-[2-(4-hydroxy-3-methoxycyclohexyl)-1-methylvinyl]-26,28-dimethoxy-13,22,24,30-tetramethyl-17-phenylmethyl-11,31-dioxa-4,17-diazatetracyclo[25.3.1.0^{4,9}.0^{16,20}]hentriaconta-16(20),18,21-trien-2,3,10-trion,
17-Allyl-1-hydroxy-12-[2-(3,4-epoxycyclohexyl)-1-methylvinyl]-23, 25-dimethoxy-13,19,21,27-tetramethyl-11,28-dioxa-4-azatricyclo[22.3.1.0^{4,9}]octacosa-14,18-dien-2,3,10,16-tetraon,
17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon-C16-oxim,
17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon-C16-oxim-O-methylether,
17-Allyl-1,14-dihydroxy-12-[2-(4-(2',5'-dioxahexyloxy)-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-Propyl-1-hydroxy-12-[2-(3,4-dihydroxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
1,14-Dihydroxy-12-[2-(4-hydroxy-3-methoxycyclohexyl)-1-methylvinyl]-23,25-dimethoxy-13,19,21,27-tetramethyl-17-(2-oxopropyl)-11,28-dioxa-4-azatricyclo[22.3.1.0^{4,9}]octacos-18-en-2,3,10,16-tetraon,
17-Allyl-1,14-dihydroxy-12-[2-(4-hydroxy-3-methoxycyclohexyl)-1-methylvinyl]-23,25-dimethoxy-13,19,21,27-tetramethy1-11,28-dioxa-4-azaspiro-[tricyclo[22.3.1.0^{4,9}]octacos-18-en-2,2'-oxiran]-3,10,16-trion,
17-Ethanalyl-1,2,14-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-3,10,16-trion,
1,14-Dihydroxy-12-[2-(4-hydroxy-3-methoxycyclohexyl)-1-methylvinyl]-26,28-dimethoxy-18-[(phenylseleno)-methyl]-13,22,24,30-tetramethyl-11,17,31-trioxa-4-azatetracyclo[25.3.1.0^{4,9}.0^{16,20}]-hentriaconta-16(20),21-dien-2,3,10-trion,
4'-Methyl-[17-allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10-trion-16-yliden]-benzolsulfonsäurehydrazid,
oder ein pharmazeutisch annehmbares Salz irgendeiner hievon.

7. 17-Propyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-Allyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
oder ein pharmazeutisch annehmbares Salz hievon.

8. Verwendung einer Verbindung der Formel (I), wie in Anspruch 1 definiert, oder einer Verbindung nach Anspruch 7, oder eines pharmazeutisch annehmbaren Salzes hievon, als Pharmazeutikum.

9. Pharmazeutische Formulierung, welche eine Verbindung der Formel (I), wie in Anspruch 1 definiert, oder eine Verbindung nach Anspruch 7, oder ein pharmazeutisch annehmbares Salz hievon, in Mischung mit einem pharmazeutisch annehmbaren Adjuvans, Verdünnungsmittel oder Träger umfaßt.

10. Verwendung einer Verbindung der Formel (I), wie in Anspruch definiert, oder einer Verbindung nach Anspruch 7, oder eines pharmazeutisch annehmbaren Salzes hievon, bei der Herstellung eines Medikaments zur Verwendung als Immunsuppressivum.

11. Verfahren zur Herstellung einer Verbindung der Formel (I), wie in Anspruch 1 definiert, oder einer Verbindung nach Anspruch 7, oder eines pharmazeutisch annehmbaren Salzes hievon, welches Verfahren umfaßt:
(a) Herstellen einer Verbindung der Formel (I), worin eines oder mehrere von [R¹ und R²], [R³ und R⁴] oder [R⁵ und R⁶] zwei benachbarte Wasserstoffatome darstellen, durch selektive Reduktion einer entsprechenden Gruppe [R¹ und R²], [R³ und R⁴] oder [R⁵ und R⁶], wenn sie eine zweite Bindung zwischen zwei benachbarten Kohlenstoffatomen in einer entsprechenden Verbindung bedeutet;
(b) Herstellen einer Verbindung der Formel (I), welche eine oder mehrere Hydroxy-Gruppen enthält, durch selektive Reduktion einer oder mehrerer C=O Gruppen in einer entsprechenden Verbindung;
(c) Herstellen einer Verbindung der Formel (I), welche eine 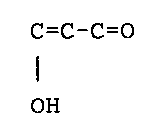 Gruppe enthält, durch selektive Oxidation einer 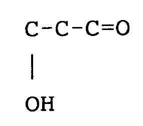 Gruppe in einer entsprechenden Verbindung;
(d) Herstellen einer Verbindung der Formel (I), welche eine oder mehrere Alkoxy-Gruppen enthält, durch Alkylierung einer oder mehrerer Hydroxy-Gruppen in einer entsprechenden Verbindung durch Umsetzen mit einem geeigneten Alkylierungsmittel;
(e) Herstellen einer Verbindung der Formel (I), welche eine oder mehrere Hydroxyl-Gruppen enthält, durch Entschützen einer oder mehrerer geschützter Hydroxy-Gruppen in einer entsprechenden Verbindung, wobei die Schutzgruppe vorzugsweise durch Hydrogenolyse entfernbar ist;
(f) Herstellen einer Verbindung der Formel (I), welche eine Kohlenstoff-Kohlenstoff-Doppelbindung enthält, durch Eliminierung von HL aus einer entsprechenden Verbindung, worin L eine Abgangsgruppe bedeutet;
(g) Herstellen einer Verbindung der Formel (I), worin Y, R¹⁰ und R²³ gemeinsam mit den Kohlenstoffatomen, an die sie gebunden sind, einen Tetrahydrofuranring, substituiert durch eine CH₂Se(C₆H₅) Gruppe, bilden, durch Umsetzen eines Phenylselenylhalogenids mit einer entsprechenden Verbindung, worin Y die Bedeutung O hat, und R¹⁰ Allyl darstellt;
(h) Herstellen einer Verbindung der Formel (I), welche einen Allylalkohol enthält, durch selektive Oxidation einer Allyl-Gruppe in einer entsprechenden Verbindung;
(i) Herstellen einer Verbindung der Formel (I), welche eine Keton-Gruppe enthält, durch Oxidation einer Hydroxy-Gruppe in einer entsprechenden Verbindung;
(j) Herstellen einer Verbindung der Formel (I), welche ein vicinales Diol enthält, durch Oxidation einer Kohlenstoff-Kohlenstoff-Doppelbindung in einer entsprechenden Verbindung,
(k) Herstellen einer Verbindung der Formel (I), welche eine Aldehyd-Gruppe enthält, durch oxidative Spaltung eines vicinalen Diols in einer entsprechenden Verbindung;
(l) Herstellen einer Verbindung der Formel (I), worin Y, R¹⁰ und R²³ gemeinsam mit den Kohlenstoffatomen, an die sie gebunden sind, einen Pyrrolring bilden, durch Umsetzen von Ammoniak oder eines Amins mit einer entsprechenden Verbindung, worin Y die Bedeutung O hat, und R¹⁰ -CH₂CHO ist;
(m) Herstellen einer Verbindung der Formel (I), welche eine Epoxid-Gruppe enthält, durch Cyclisierung einer 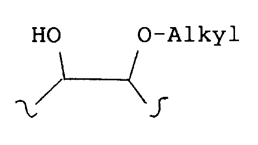 Gruppe in einer entsprechenden Verbindung;
(n) Herstellen einer Verbindung der Formel (I), worin Y eine Oxim-Gruppe darstellt, durch Umsetzen einer entsprechenden Verbindung, worin Y die Bedeutung O hat, mit einem Sauerstoffsubstituierten Amin;
(o) Herstellen einer Verbindung der Formel (I), welche eine COCH₃ Gruppe enthält, durch Oxidation einer Alken-Endgruppe in einer entsprechenden Verbindung;
(p) Herstellen einer Verbindung der Formel (I), worin X -CH₂O- darstellt, durch Umsetzen einer entsprechenden Verbindung, worin X die Bedeutung O hat, mit Diazomethan, oder
(q) Herstellen einer Verbindung der Formel (I), worin Y ein Hydrazon oder ein Hydrazon-Derivat darstellt, durch Umsetzen einer entsprechenden Verbindung, worin Y die Bedeutung O hat, mit Hydrazin oder einem substituierten Hydrazin.

## Patentansprüche (Patentansprüche für folgende(n) Vertragsstaat(en): AT, BE, LU, SE)

1. Verbindung der Formel (I) 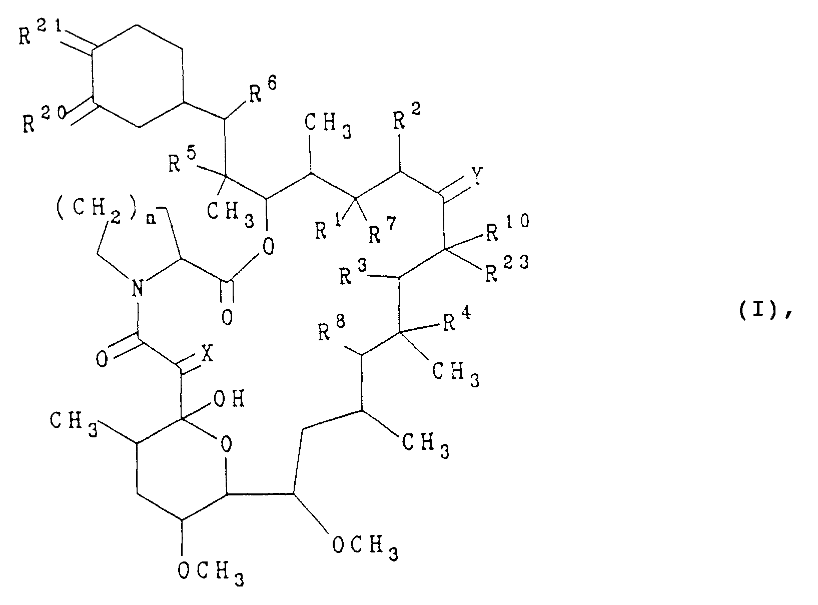 worin jedes benachbarte Substituentenpaar [R¹ und R²],
[R³ und R⁴] und [R⁵ und R⁶] unabhängig:
a) zwei benachbarte Wasserstoffatome darstellt, oder
b) eine zweite Bindung zwischen den benachbarten Kohlenstoffatomen, an die sie gebunden sind, bildet;
R⁷ die Bedeutung H, OH oder O-C₁-C₆-Alkyl hat, oder zusammen mit R¹ =O darstellen kann;
R⁸ die Bedeutung H oder OH hat;
R¹⁰ C₁-C₆-Alkyl, C₁-C₆-Alkyl substituiert durch eine oder mehrere Hydroxy-Gruppen, C₂-C₆-Alkenyl, C₂-C₆-Alkenyl substituiert durch eine oder mehrere Hydroxy-Gruppen, oder C₁-C₆-Alkyl substituiert durch =O ist;
X die Bedeutung O, (H,OH) oder -CH₂O- hat;
Y die Bedeutung O, (H,OH), N-NR¹¹R¹² oder N-OR¹³ hat;
R¹¹ und R¹² unabhängig H, C₁-C₆-Alkyl, Phenyl oder Tosyl sind;
R¹³ die Bedeutung H oder C₁-C₆-Alkyl hat;
R²⁰ und R²¹ unabhängig O darstellen, oder unabhängig (R²⁰a,H) bzw. (R²¹a,H) sein können; R²⁰a und R²¹a unabhängig OH, O-C₁-C₆-Alkyl oder OCH₂OCH₂CH₂OCH₃ darstellen; außerdem R²⁰a und R²¹a gemeinsam ein Sauerstoffatom in einem Epoxidring bedeuten können;
R²³ H darstellt;
n 1 oder 2 ist;
zusätzlich zu ihren obigen Bedeutungen Y, R¹⁰ und R²³ gemeinsam mit den Kohlenstoffatomen, an die sie gebunden sind, darstellen können: einen 5- oder 6-gliedrigen, N-, S- oder O-haltigen heterocyclischen Ring, der gesättigt oder ungesättigt sein kann, und der substituiert sein kann durch eine oder mehrere Gruppen, ausgewählt aus C₁-C₆-Alkyl, Hydroxy, C₁-C₆-Alkyl substituiert durch eine oder mehrere Hydroxy-Gruppen, O-C₁-C₆-Alkyl, Benzyl und -CH₂Se(C₆H₅);
vorausgesetzt, daß:
(a) wenn [R³ und R⁴] und [R⁵ und R⁶] jeweils eine Kohlenstoff-Kohlenstoff-Bindung darstellen; R⁷ H oder eine OH Gruppe bedeutet; R⁸ H ist; R¹⁰ Methyl, Ethyl, Propyl oder Allyl bedeutet; R²⁰a OCH₃ darstellt; und R²¹a OH ist; X und Y nicht beide O sind; und
(b) wenn [R¹ und R²] zwei benachbarte Wasserstoffatome darstellt; [R³ und R⁴] und [R⁵ und R⁶] jeweils eine Kohlenstoff-Kohlenstoff-Bindung sind; R⁷ eine OH Gruppe bedeutet; R⁸ H ist; R¹⁰ Allyl bedeutet; R²¹a OH ist; X und Y beide O sind; und n 2 ist; R²⁰ nicht O oder (OH,H) darstellt;
und pharmazeutisch annehmbare Salze hievon.

2. Verbindung nach Anspruch 1, worin R¹⁰ Methyl, Ethyl, Propyl oder Allyl bedeutet.

3. Verbindung nach Anspruch 1 oder 2, worin zumindest eines von R²⁰a und R²¹a die Bedeutung OH oder OCH₃ hat.

4. Verbindung nach einem der vorhergehenden Ansprüche, worin n 2 ist.

5. Verbindung nach einem der vorhergehenden Ansprüche, worin R⁷ H oder OH darstellt.

6. Verbindung nach Anspruch 1, nämlich:
17-Allyl-1-hydroxy-12-[2-(3,4-dimethoxycyclohexyl)-1-methylvinyl]-14,23,25-trimethoxy-13,19,21,27-tetramethyl-11,28-dioxa-4-azatricyclo[22.3.1.0^{4,9}]octacos-18-en-2,3,10,16-tetraon,
17-Allyl-1,14-dihydroxy-12-[2-(3,4-dimethoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
1,14-Dihydroxy-12-[2-(4-hydroxy-3-methoxycyclohexyl)-1-methylvinyl]-18-[(phenylseleno)-methyl]-16,26,28-trimethoxy-[ 13,22,24,30-tetramethyl-11,17,31-trioxa-4-azatetracyclo-25.3.1.0^{4,9}.0^{16,20}]hentriacont-21-en-2,3,10-trion,
17-Allyl-1-hydroxy-12-[2-(3,4-dimethoxycyclohexyl)-1-methylvinyl]-23,25-dimethoxy-13,19,21,27-tetramethyl-11,28-dioxa-4-azatricyclo[22.3.1.0^{4,9}]octacosa-14,18-dien-2,3,10,16-tetraon,
17-Allyl-1,2,14-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-3,10,16-trion,
17-Allyl-1,2,14,16-tetrahydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-3,10-dion,
17-Propyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacosan-2,3,10,16-tetraon,
17-Propyl-1,14-dihydroxy-12-[2-(4-hydroxy-3-methoxycyclohexyl)-1-methylethyl]-23,25-dimethoxy-13,19,21,27-tetramethyl-11,28-dioxa-4-azatricyclo[22.3.1.0^{4,9}]octacos-18-en-2,3,10,16-tetraon,
17-Propyl-1,14-dihydroxy-12-[2-(4-hydroxy-3-methoxycyclohexyl)-1-methylethyl]-23,25-dimethoxy-13,19,21,27-tetramethyl-11,28-dioxa-4-azatricyclo[22.3.1.0^{4,9}]octacosan-2,3,10,16-tetraon,
17-Allyl-1,14,20-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-(1-Hydroxyprop-2-enyl)-1,14,20-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-Allyl-1,2-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-3,10,16-trion,
17-Allyl-1,16-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacosa-14,18-dien-2,3,10-trion,
17-Propyl-1-hydroxy-12-[2-(3-methoxy-4-oxocyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-(2,3-Dihydroxypropyl)-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-Ethanalyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
1,14-Dihydroxy-12-[2-(4-hydroxy-3-methoxycyclohexyl)-1-methylvinyl]-26,28-dimethoxy-13,22,24,30-tetramethyl-11,31-dioxa-4,17-diazatetracyclo[25.3.1.0^{4,9}.0^{16,20}]hentriaconta-16(20),18,21-trien-2,3,10-trion,
1,14-Dihydroxy-12-[2-(4-hydroxy-3-methoxycyclohexyl)-1-methylvinyl]-26,28-dimethoxy-17-(2-hydroxyethyl)-13,22,24,30-tetramethyl-11,31-dioxa-4,17-diazatetracyclo[25.3.1.0^{4,9}.0^{16,20}]hentriaconta-16(20),18,21-trien-2,3,10-trion,
1,14-Dihydroxy-12-[2-(4-hydroxy-3-methoxycyclohexyl)-1-methylvinyl]-26,28-dimethoxy-13,22,24,30-tetramethyl-17-phenylmethyl-11,31-dioxa-4,17-diazatetracyclo[25.3.1.0^{4,9}.0^{16,20}]hentriaconta-16(20),18,21-trien-2,3,10-trion,
17-Allyl-1-hydroxy-12-[2-(3,4-epoxycyclohexyl)-1-methylvinyl]-23 ,25-dimethoxy-13,19,21,27-tetramethyl-11,28-dioxa-4-azatricyclo[22.3.1.0^{4,9}]octacosa-14,18-dien-2,3,10,16-tetraon,
17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon-C16-oxim,
17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon-C16-oxim-O-methylether,
17-Allyl-1,14-dihydroxy-12-[2-(4-(2',5'-dioxahexyloxy)-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-Propyl-1-hydroxy-12-[2-(3,4-dihydroxycyclohexyl)-1-methylvinyl]-23,25-dimethoxy-13,19,21,27-tetramethyl-11,28-dioxa-4- azatricyclo[22.3.1.0^{4,9}]octacos-18-en-2,3,10,16-tetraon,
1,14-Dihydroxy-12-[2-(4-hydroxy-3-methoxycyclohexyl)-1-methylvinyl]-23,25-dimethoxy-13,19,21,27-tetramethyl-17-(2-oxopropyl)-11,28-dioxa-4-azatricyclo[22.3.1.0^{4,9}]octacos-18-en-2,3,10,16-tetraon,
17-Allyl-1,14-dihydroxy-12-[2-(4-hydroxy-3-methoxycyclohexyl)-1-methylvinyl]-23,25-dimethoxy-13,19,21,27-tetramethy1-11,28-dioxa-4-azaspiro-[tricyclo[22.3.1.0^{4,9}]octacos-18-en-2,2'-oxiran]-3,10,16-trion,
17-Ethanalyl-1,2,14-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-3,10,16-trion,
1,14-Dihydroxy-12-[2-(4-hydroxy-3-methoxycyclohexyl)-1-methylvinyl]-26,28-dimethoxy-18-[(phenylseleno)-methyl]-13,22,24,30-tetramethyl-11,17,31-trioxa-4-azatetracyclo[25.3.1.0^{4,9}.0^{16,20}]hentriaconta-16(20),21-dien-2,3,10-trion,
4'-Methyl-[17-allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10-trion-16-yliden]-benzolsulfonsäurehydrazid,
oder ein pharmazeutisch annehmbares Salz irgendeiner hievon.

7. 17-Propyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-Allyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
oder ein pharmazeutisch annehmbares Salz hievon.

8. Verwendung einer Verbindung der Formel (I), wie in Anspruch 1 definiert, oder einer Verbindung nach Anspruch 7, oder eines pharmazeutisch annehmbaren Salzes hievon, als Pharmazeutikum.

9. Pharmazeutische Formulierung, welche eine Verbindung der Formel (I), wie in Anspruch 1 definiert, oder eine Verbindung nach Anspruch 7, oder ein pharmazeutisch annehmbares Salz hievon, in Mischung mit einem pharmazeutisch annehmbaren Adjuvans, Verdünnungsmittel oder Träger umfaßt.

10. Verwendung einer Verbindung der Formel (I), wie in Anspruch 1 definiert, oder einer Verbindung nach Anspruch 7, oder eines pharmazeutisch annehmbaren Salzes hievon, bei der Herstellung eines Medikaments zur Verwendung als Immunsuppressivum.

11. Verfahren zur Herstellung einer Verbindung der Formel (I), wie in Anspruch 1 definiert, oder einer Verbindung nach Anspruch 7, oder eines pharmazeutisch annehmbaren Salzes hievon, welches umfaßt:
(a) Herstellen einer Verbindung der Formel (I), worin eines oder mehrere von [R¹ und R²], [R³ und R⁴] oder [R⁵ und R⁶] zwei benachbarte Wasserstoffatome darstellen, durch selektive Reduktion einer entsprechenden Gruppe [R¹ und R²], [R³ und R⁴] oder [R⁵ und R⁶], wenn sie eine zweite Bindung zwischen zwei benachbarten Kohlenstoffatomen in einer entsprechenden Verbindung bedeutet;
(b) Herstellen einer Verbindung der Formel (I), welche eine oder mehrere Hydroxy-Gruppen enthält, durch selektive Reduktion einer oder mehrerer C=O Gruppen in einer entsprechenden Verbindung;
(c) Herstellen einer Verbindung der Formel (I), welche eine 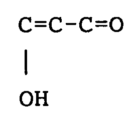 Gruppe enthält, durch selektive Oxidation einer 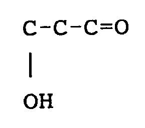 Gruppe in einer entsprechenden Verbindung;
(d) Herstellen einer Verbindung der Formel (I), welche eine oder mehrere Alkoxy-Gruppen enthält, durch Alkylierung einer oder mehrerer Hydroxy-Gruppen in einer entsprechenden Verbindung durch Umsetzen mit einem geeigneten Alkylierungsmittel;
(e) Herstellen einer Verbindung der Formel (I), welche eine oder mehrere Hydroxyl-Gruppen enthält, durch Entschützen einer oder mehrerer geschützter Hydroxy-Gruppen in einer entsprechenden Verbindung, wobei die Schutzgruppe vorzugsweise durch Hydrogenolyse entfernbar ist;
(f) Herstellen einer Verbindung der Formel (I), welche eine Kohlenstoff-Kohlenstoff-Doppelbindung enthält, durch Eliminierung von HL aus einer entsprechenden Verbindung, worin L eine Abgangsgruppe bedeutet;
(g) Herstellen einer Verbindung der Formel (I), worin Y, R¹⁰ und R²³ gemeinsam mit den Kohlenstoffatomen, an die sie gebunden sind, einen Tetrahydrofuranring, substituiert durch eine CH₂Se(C₆H₅) Gruppe, bilden, durch Umsetzen eines Phenylselenylhalogenids mit einer entsprechenden Verbindung, worin Y die Bedeutung O hat, und R¹⁰ Allyl darstellt;
(h) Herstellen einer Verbindung der Formel (I), welche einen Allylalkohol enthält, durch selektive Oxidation einer Allyl-Gruppe in einer entsprechenden Verbindung;
(i) Herstellen einer Verbindung der Formel (I), welche eine Keton-Gruppe enthält, durch Oxidation einer Hydroxy-Gruppe in einer entsprechenden Verbindung;
(j) Herstellen einer Verbindung der Formel (I), welche ein vicinales Diol enthält, durch Oxidation einer Kohlenstoff-Kohlenstoff-Doppelbindung in einer entsprechenden Verbindung,
(k) Herstellen einer Verbindung der Formel (I), welche eine Aldehyd-Gruppe enthält, durch oxidative Spaltung eines vicinalen Diols in einer entsprechenden Verbindung;
(l) Herstellen einer Verbindung der Formel (I), worin Y, R¹⁰ und R²³ gemeinsam mit den Kohlenstoffatomen, an die sie gebunden sind, einen Pyrrolring bilden, durch Umsetzen von Ammoniak oder eines Amins mit einer entsprechenden Verbindung, worin Y die Bedeutung O hat, und R¹⁰ -CH₂CHO ist;
(m) Herstellen einer Verbindung der Formel (I), welche eine Epoxid-Gruppe enthält, durch Cyclisierung einer 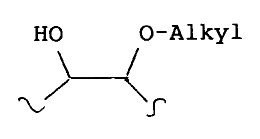 Gruppe in einer entsprechenden Verbindung;
(n) Herstellen einer Verbindung der Formel (I), worin Y eine Oxim-Gruppe darstellt, durch Umsetzen einer entsprechenden Verbindung, worin Y die Bedeutung O hat, mit einem Sauerstoffsubstituierten Amin;
(o) Herstellen einer Verbindung der Formel (I), welche eine COCH₃ Gruppe enthält, durch Oxidation einer Alken-Endgruppe in einer entsprechenden Verbindung;
(p) Herstellen einer Verbindung der Formel (I), worin X -CH₂O- darstellt, durch Umsetzen einer entsprechenden Verbindung, worin X die Bedeutung O hat, mit Diazomethan, oder
(q) Herstellen einer Verbindung der Formel (I), worin Y ein Hydrazon oder ein Hydrazon-Derivat darstellt, durch Umsetzen einer entsprechenden Verbindung, worin Y die Bedeutung O hat, mit Hydrazin oder einem substituierten Hydrazin.

## Patentansprüche (Patentansprüche für folgende(n) Vertragsstaat(en): ES, GR)

1. Verfahren zur Herstellung einer Verbindung der Formel (I) 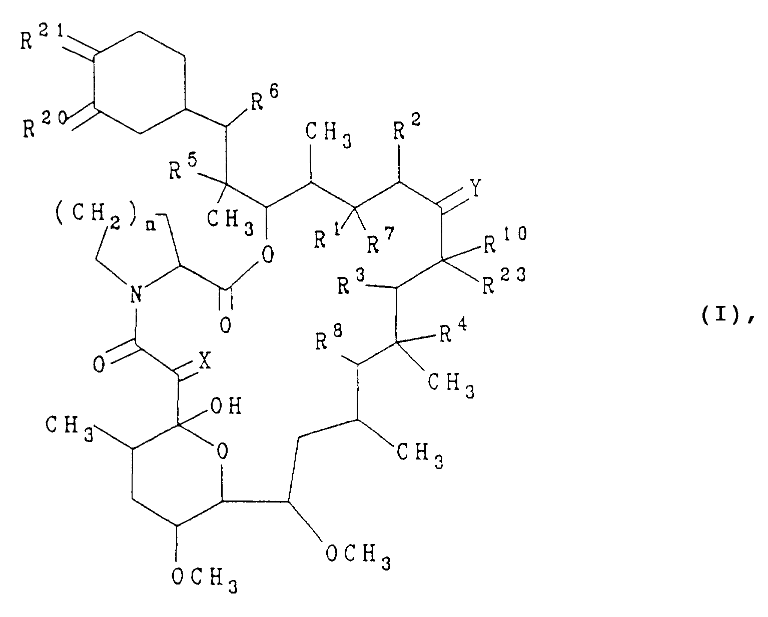 worin jedes benachbarte Substituentenpaar [R¹ und R²],
[R³ und R⁴] und [R⁵ und R⁶] unabhängig:
a) zwei benachbarte Wasserstoffatome darstellt, oder
b) eine zweite Bindung zwischen den benachbarten Kohlenstoffatomen, an die sie gebunden sind, bildet;
R⁷ die Bedeutung H, OH oder O-C₁-C₆-Alkyl hat, oder zusammen mit R¹ =O darstellen kann;
R⁸ die Bedeutung H oder OH hat;
R¹⁰ C₁-C₆-Alkyl, C₁-C₆-Alkyl substituiert durch eine oder mehrere Hydroxy-Gruppen, C₂-C₆-Alkenyl, C₂-C₆-Alkenyl substituiert durch eine oder mehrere Hydroxy-Gruppen, oder C₁-C₆-Alkyl substituiert durch =O ist;
X die Bedeutung O, (H,OH) oder -CH₂O- hat;
Y die Bedeutung O, (H,OH), N-NR¹¹R¹² oder N-OR¹³ hat;
R¹¹ und R¹² unabhängig H, C₁-C₆-Alkyl, Phenyl oder Tosyl sind;
R¹³ die Bedeutung H oder C₁-C₆-Alkyl hat;
R²⁰ und R²¹ unabhängig O darstellen, oder unabhängig (R²⁰a,H) bzw. (R²¹a,H) sein können; R²⁰a und R²¹a unabhängig OH, O-C₁-C₆-Alkyl oder OCH₂OCH₂CH₂OCH₃ darstellen; außerdem R²⁰a und R²¹a gemeinsam ein Sauerstoffatom in einem Epoxidring bedeuten können;
R²³ H darstellt;
n 1 oder 2 ist;
zusätzlich zu ihren obigen Bedeutungen Y, R¹⁰ und R²³ gemeinsam mit den Kohlenstoffatomen, an die sie gebunden sind, darstellen können: einen 5- oder 6-gliedrigen, N-, S- oder O-haltigen heterocyclischen Ring, der gesättigt oder ungesättigt sein kann, und der substituiert sein kann durch eine oder mehrere Gruppen, ausgewählt aus C₁-C₆-Alkyl, Hydroxy, C₁-C₆-Alkyl substituiert durch eine oder mehrere Hydroxy-Gruppen, O-C₁-C₆-Alkyl, Benzyl und -CH₂Se(C₆H₅);
vorausgesetzt, daß:
(a) wenn [R³ und R⁴] und [R⁵ und R⁶] jeweils eine Kohlenstoff-Kohlenstoff-Bindung darstellen; R⁷ H oder eine OH Gruppe bedeutet; R⁸ H ist; R¹⁰ Methyl, Ethyl, Propyl oder Allyl bedeutet; R²⁰a OCH₃ darstellt; und R²¹a OH ist; X und Y nicht beide O sind; und
(b) wenn [R¹ und R²] zwei benachbarte Wasserstoffatome darstellt; [R³ und R⁴] und [R⁵ und R⁶] jeweils eine Kohlenstoff-Kohlenstoff-Bindung sind; R⁷ eine OH Gruppe bedeutet; R⁸ H ist; R¹⁰ Allyl bedeutet; R²¹a OH ist; X und Y beide O sind; und n 2 ist; R²⁰ nicht O oder (OH,H) darstellt;
und pharmazeutisch annehmbarer Salze hievon, welches Verfahren umfaßt:
(a) Herstellen einer Verbindung der Formel (I), worin eines oder mehrere von [R¹ und R²], [R³ und R⁴] oder [R⁵ und R⁶] zwei benachbarte Wasserstoffatome darstellen, durch selektive Reduktion einer entsprechenden Gruppe [R¹ und R²], [R³ und R⁴] oder [R⁵ und R⁶], wenn sie eine zweite Bindung zwischen zwei benachbarten Kohlenstoffatomen in einer entsprechenden Verbindung bedeutet;
(b) Herstellen einer Verbindung der Formel (I), welche eine oder mehrere Hydroxy-Gruppen enthält, durch selektive Reduktion einer oder mehrerer C=O Gruppen in einer entsprechenden Verbindung;
(c) Herstellen einer Verbindung der Formel (I), welche eine 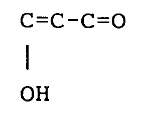 Gruppe enthält, durch selektive Oxidation einer 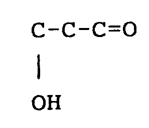 Gruppe in einer entsprechenden Verbindung;
(d) Herstellen einer Verbindung der Formel (I), welche eine oder mehrere Alkoxy-Gruppen enthält, durch Alkylierung einer oder mehrerer Hydroxy-Gruppen in einer entsprechenden Verbindung durch Umsetzen mit einem geeigneten Alkylierungsmittel;
(e) Herstellen einer Verbindung der Formel (I), welche eine oder mehrere Hydroxyl-Gruppen enthält, durch Entschützen einer oder mehrerer geschützter Hydroxy-Gruppen in einer entsprechenden Verbindung, wobei die Schutzgruppe vorzugsweise durch Hydrogenolyse entfernbar ist;
(f) Herstellen einer Verbindung der Formel (I), welche eine Kohlenstoff-Kohlenstoff-Doppelbindung enthält, durch Eliminierung von HL aus einer entsprechenden Verbindung, worin L eine Abgangsgruppe bedeutet;
(g) Herstellen einer Verbindung der Formel (I), worin Y, R¹⁰ und R²³ gemeinsam mit den Kohlenstoffatomen, an die sie gebunden sind, einen Tetrahydrofuranring, substituiert durch eine CH₂Se(C₆H₅) Gruppe, bilden, durch Umsetzen eines Phenylselenylhalogenids mit einer entsprechenden Verbindung, worin Y die Bedeutung O hat, und R¹⁰ Allyl darstellt;
(h) Herstellen einer Verbindung der Formel (I), welche einen Allylalkohol enthält, durch selektive Oxidation einer Allyl-Gruppe in einer entsprechenden Verbindung;
(i) Herstellen einer Verbindung der Formel (I), welche eine Keton-Gruppe enthält, durch Oxidation einer Hydroxy-Gruppe in einer entsprechenden Verbindung;
(j) Herstellen einer Verbindung der Formel (I), welche ein vicinales Diol enthält, durch Oxidation einer Kohlenstoff-Kohlenstoff-Doppelbindung in einer entsprechenden Verbindung,
(k) Herstellen einer Verbindung der Formel (I), welche eine Aldehyd-Gruppe enthält, durch oxidative Spaltung eines vicinalen Diols in einer entsprechenden Verbindung;
(l) Herstellen einer Verbindung der Formel (I), worin Y, R¹⁰ und R²³ gemeinsam mit den Kohlenstoffatomen, an die sie gebunden sind, einen Pyrrolring bilden, durch Umsetzen von Ammoniak oder eines Amins mit einer entsprechenden Verbindung, worin Y die Bedeutung O hat, und R¹⁰ -CH₂CHO ist;
(m) Herstellen einer Verbindung der Formel (I), welche eine Epoxid-Gruppe enthält, durch Cyclisierung einer 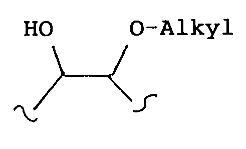 Gruppe in einer entsprechenden Verbindung;
(n) Herstellen einer Verbindung der Formel (I), worin Y eine Oxim-Gruppe darstellt, durch Umsetzen einer entsprechenden Verbindung, worin Y die Bedeutung O hat, mit einem Sauerstoffsubstituierten Amin;
(o) Herstellen einer Verbindung der Formel (I), welche eine COCH₃ Gruppe enthält, durch Oxidation einer Alken-Endgruppe in einer entsprechenden Verbindung;
(p) Herstellen einer Verbindung der Formel (I), worin X -CH₂O- darstellt, durch Umsetzen einer entsprechenden Verbindung, worin X die Bedeutung O hat, mit Diazomethan, oder
(q) Herstellen einer Verbindung der Formel (I), worin Y ein Hydrazon oder ein Hydrazon-Derivat darstellt, durch Umsetzen einer entsprechenden Verbindung, worin Y die Bedeutung O hat, mit Hydrazin oder einem substituierten Hydrazin.

2. Verfahren nach Anspruch 1, wobei R¹⁰ Methyl, Ethyl, Propyl oder Allyl bedeutet.

3. Verfahren nach Anspruch 1 oder 2, wobei zumindest eines von R²⁰a und R²¹a die Bedeutung OH oder OCH₃ hat.

4. Verfahren nach einem der vorhergehenden Ansprüche, wobei n 2 ist.

5. Verfahren nach einem der vorhergehenden Ansprüche, wobei R⁷ H oder OH darstellt.

6. Verfahren nach Anspruch 1, bei welchem die Verbindung der Formel (I):
17-Allyl-1-hydroxy-12-[2-(3,4-dimethoxycyclohexyl)-1-methylvinyl]-14,23,25-trimethoxy-13,19,21,27-tetramethyl-11,28-dioxa-4-azatricyclo[22.3.1.0^{4,9}]octacos-18-en-2,3,10,16-tetraon,
17-Allyl-1,14-dihydroxy-12-[2-(3,4-dimethoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
1,14-Dihydroxy-12-[2-(4-hydroxy-3-methoxycyclohexyl)-1-methylvinyl]-18-[(phenylseleno)-methyl]-16,26,28-trimethoxy-13,22,24,30-tetramethyl-11,17,31-trioxa-4-azatetracyclo-] 25.3.1.0^{4,9}.0^{16,20}]hentriacont-21-en-2,3,10-trion,
17-Allyl-1-hydroxy-12-[2-(3,4-dimethoxycyclohexyl)-1-methylvinyl]-23,25-dimethoxy-13,19,21,27-tetramethyl-11,28-dioxa-4-azatricyclo[22.3.1.0^{4,9}]octacosa-14,18-dien-2,3,10,16-tetraon,
17-Allyl-1,2,14-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-3,10,16-trion,
17-Allyl-1,2,14,16-tetrahydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-3,10-dion,
17-Propyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacosan-2,3,10,16-tetraon,
17-Propyl-1,14-dihydroxy-12-[2-(4-hydroxy-3-methoxycyclohexyl)-1-methylethyl]-23,25-dimethoxy-13,19,21,27-tetramethyl-11,28-dioxa-4-azatricyclo[22.3.1.0^{4,9}]octacos-18-en-2,3,10,16-tetraon,
17-Propyl-1,14-dihydroxy-12-[2-(4-hydroxy-3-methoxycyclohexyl)-1-methylethyl]-23,25-dimethoxy-13,19,21,27-tetramethyl-11,28-dioxa-4-azatricyclo[22.3.1.0^{4,9}]octacosan-2,3,10,16-tetraon,
17-Allyl-1,14,20-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-(1-Hydroxyprop-2-enyl)-1,14,20-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-Allyl-1,2-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-3,10,16-trion,
17-Allyl-1,16-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacosa-14,18-dien-2,3,10-trion,
17-Propyl-1-hydroxy-12-[2-(3-methoxy-4-oxocyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-(2,3-Dihydroxypropyl)-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-Ethanalyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
1,14-Dihydroxy-12-[2-(4-hydroxy-3-methoxycyclohexyl)-1-methylvinyl]-26,28-dimethoxy-13,22,24,30-tetramethyl-11,31-dioxa-4,17-diazatetracyclo[25.3.1.0^{4,9}.0^{16,20}]hentriaconta-16(20),18,21-trien-2,3,10-trion,
1,14-Dihydroxy-12-[2-(4-hydroxy-3-methoxycyclohexyl)-1-methylvinyl]-26,28-dimethoxy-17-(2-hydroxyethyl)-13,22,24,30-tetramethyl-11,31-dioxa-4,17-diazatetracyclo[25.3.1.0^{4,9}.0^{16,20}]hentriaconta-16(20),18,21-trien-2,3,10-trion,
1,14-Dihydroxy-12-[2-(4-hydroxy-3-methoxycyclohexyl)-1-methylvinyl]-26,28-dimethoxy-13,22,24,30-tetramethyl-17-phenylmethyl-11,31-dioxa-4,17-diazatetracyclo[25.3.1.0^{4,9}.0^{16,20}]hentriaconta-16(20),18,21-trien-2,3,10-trion,
17-Allyl-1-hydroxy-12-[2-(3,4-epoxycyclohexyl)-1-methylvinyl]-23, 25-dimethoxy-13,19,21,27-tetramethyl-11,28-dioxa-4-azatricyclo[22.3.1.0^{4,9}]octacosa-14,18-dien-2,3,10,16-tetraon,
17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon-C16-oxim,
17-Allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon-C16-oxim-O-methylether,
17-Allyl-1,14-dihydroxy-12-[2-(4-(2',5'-dioxahexyloxy)-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-Propyl-1-hydroxy-12-[2-(3,4-dihydroxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
1,14-Dihydroxy-12-[2-(4-hydroxy-3-methoxycyclohexyl)-1-methylvinyl]-23,25-dimethoxy-13,19,21,27-tetramethyl-17-(2-oxopropyl)-11,28-dioxa-4-azatricyclo[22.3.1.0^{4,9}]octacos-18-en-2,3,10,16-tetraon,
17-Allyl-1,14-dihydroxy-12-[2-(4-hydroxy-3-methoxycyclohexyl)-1-methylvinyl]-23,25-dimethoxy-13,19,21,27-tetramethy1-11,28-dioxa-4-azaspiro-[tricyclo[22.3.1.0^{4,9}]octacos-18-en-2,2'-oxiran]-3,10,16-trion,
17-Ethanalyl-1,2,14-trihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-3,10,16-trion,
1,14-Dihydroxy-12-[2-(4-hydroxy-3-methoxycyclohexyl)-1-methylvinyl]-26,28-dimethoxy-18-[(phenylseleno)-methyl]-13,22,24,30-tetramethyl-11,17,31-trioxa-4-azatetracyclo[25.3.1.0^{4,9}.0^{16,20}]hentriaconta-16(20),21-dien-2,3,10-trion,
4'-Methyl-[17-allyl-1,14-dihydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacsos-18-en-2,3,10-trion-16-yliden]-benzolsulfonsäurehydrazid,
oder ein pharmazeutisch annehmbares Salz irgendeiner hievon ist.

7. Verfahren zur Herstellung von:
17-Propyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
17-Allyl-1-hydroxy-12-[2-(4-hydroxy-3-methoxycyclohexyl)-1-methylvinyl]-23,25-dimethoxy-13,19,21,27-tetramethyl-11,28-dioxa-4-azatricyclo[22.3.1.0^{4,9}]octacos-18-en-2,3,10,16-tetraon,
oder eines pharmazeutisch annehmbaren Salzes hievon, welches Verfahren die selektive Reduktion einer entsprechenden Verbindung, die eine Kohlenstoff-Kohlenstoff-Doppelbindung in Stellung 14 aufweist, umfaßt.

8. Verfahren zur Herstellung einer pharmazeutischen Formulierung, die als aktiven Bestandteil eine Verbindung der Formel (I), wie in Anspruch 1 definiert, oder eine Verbindung wie in Anspruch 7 definiert, oder ein pharmazeutisch annehmbares Salz hievon umfaßt, welches Verfahren das Mischen des aktiven Bestandteils mit einem pharmazeutisch annehmbaren Adjuvans, Verdünnungsmittel oder Träger umfaßt.

## Revendications (Revendications pour l'(les) Etat(s) contractant(s) suivant(s): CH, LI, DE, FR, GB, IT, NL)

1. Composé de formule (I) : 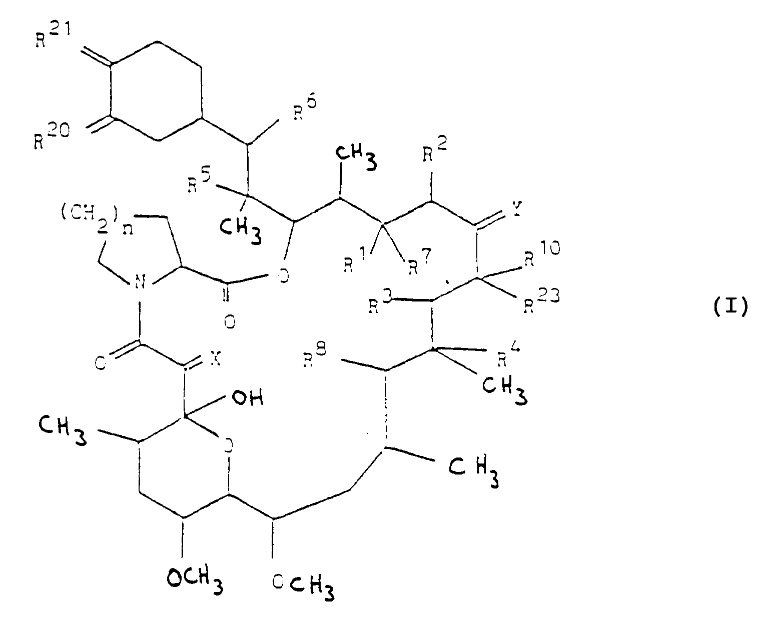 dans lequel :
chaque paire vicinale de substituants [R¹ et R²], [R³ et R⁴] et [R⁵ et R⁶], indépendamment,
a) représente deux atomes d'hydrogène vicinaux, ou
b) forme une deuxième liaison entre les atomes de carbone vicinaux auxquels ils sont fixés;
R⁷ représente H, OH, O-alcoyle en C₁₋₆ ou, en association avec R¹, peut représenter =O;
R⁸ représente H ou OH;
R¹⁰ représente alcoyle en C₁₋₆, alcoyle en C₁₋₆ substitué par un ou plusieurs radicaux hydroxyle, alcényle en C₂₋₆, alcényle en C₂₋₆ substitué par un ou plusieurs radicaux hydroxyle, ou alcoyle en C₁₋₆ substitué par =O;
X représente O, (H,OH) ou -CH₂O-;
Y représente O, (H,OH), N-NR¹¹R¹² ou N-OR¹³;
R¹¹ et R¹², indépendamment, représentent H, alcoyle en C₁₋₆, phényle ou tosyle;
R¹³ représente H ou alcoyle en C₁₋₆;
R²⁰ et R²¹, indépendamment, représentent O, ou ils peuvent représenter, indépendamment, (R²⁰a,H) et (R²¹a,H), respectivement; R²⁰a et R²¹a représentent, indépendamment, OH, O-alcoyle en C₁₋₆ ou OCH₂OCH₂CH₂OCH₃; en outre, R²⁰a et R²¹a peuvent représenter, ensemble, un atome d'oxygène dans un cycle époxyde;
R²³ représente H, et
n est 1 ou 2,
en plus de leurs significations données ci-dessus, Y, R¹⁰ et R²³, avec les atomes de carbone auxquels ils sont fixés, peuvent représenter un hétérocycle à 5 ou 6 membres, contenant N, S ou O, qui peut être saturé ou non saturé et qui peut être substitué par un ou plusieurs radicaux choisis parmi alcoyle en C₁₋₆, hydroxyle, alcolyle en C₁₋₆ substitué par un ou plusieurs radicaux hydroxyle, O-alcoyle en C₁₋₆, benzyle et CH₂Se(C₆H₅);
pourvu que :
(a) lorsque [R³ et R⁴] et [R⁵ et R⁶] représentent chacun une liaison carbone-carbone; R⁷ représente H ou un radical OH; R⁸ représente H; R¹⁰ représente méthyle, éthyle, propyle ou allyle; R²⁰a représente OCH₃, et R²¹a représente OH, alors X et Y ne représentent pas O;
(b) lorsque [R¹ et R²] représente deux atomes d'hydrogène vicinaux; [R³ et R⁴] et [R⁵ et R⁶] représentent chacun une liaison carbone-carbone; R⁷ représente un radical OH; R⁸ représente H; R¹⁰ représente allyle; R²¹a représente OH; X et Y représentent chacun O, et n est 2, alors R²⁰ ne peut pas représenter O ou (OH,H), et
(c) lorsque [R¹ et R²] représente deux atomes d'hydrogène vicinaux, [R³ et R⁴] et [R⁵ et R⁶] représentent chacun une liaison carbone-carbone; R⁷ représente un radical OH; R⁸ représente H; R¹⁰ représente éthyle; R²¹a représente OH; X et Y représentent chacun O, et n est 2, alors R²⁰a ne peut pas représenter OH;
et des sels pharmaceutiquement acceptables de celui-ci.

2. Composé suivant la revendication 1, dans lequel R¹⁰ représente méthyle, éthyle, propyle ou allyle.

3. Composé suivant la revendication 1 ou 2, dans lequel au moins un des R²⁰a et R²¹a représente OH ou OCH₃.

4. Composé suivant l'une quelconque des revendications précédentes, dans lequel n est 2.

5. Composé suivant l'une quelconque des revendications précédentes, dans lequel R⁷ représente H ou OH.

6. Composé suivant la revendication 1, qui est :
la 17-allyl-1-hydroxy-12-[2-(3,4-diméthoxycyclohexyl)-1-méthylvinyl]-14,23,25-triméthoxy-13,19,21,27-tétraméthyl-11,28-dioxa-4-azatricyclo[22.3.1.0^{4,9}]octacos-18-ène-2,3,10,16-tétraone;
la 17-allyl-1,14-dihydroxy-12-[2-(3,4-diméthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 1,14-dihydroxy-12-[2-(4-hydroxy-3-méthoxycyclohexyl)-1-méthylvinyl]-18-[(phénylséléno)méthyl]-16,26,28-triméthoxy-13,22,24,30-tétraméthyl-11,17,31-trioxa-4-azatétracyclo[25.3.1.0^{4,9}.0^{16,20}]hentriacont-21-ène-2,3,10-trione;
la 17-allyl-1-hydroxy-12-[2-(3,4-diméthoxycyclohexyl)-1-méthylvinyl]-23,25-diméthoxy-13,19,21,27-tétraméthyl-11,28-dioxa-4-azatricyclo[22.3.1.0^{4,9}]octacosa-14,18-diène-2,3,10,16-tétraone;
la 17-allyl-1,2,14-trihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-3,10,16-trione;
la 17-allyl-1,2,14,16-tétrahydroxy-12-[2-(4-hydroxy-3-méthoxycyclohexyl)-1-méthylvinyl]-23,25-diméthoxy- 13,19,21,27-tétraméthyl-11,28-dioxa-4-azatricyclo[22.3.1.0^{4,9}]octacos-18-ène-3,10-dione;
la 17-propyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacosane-2,3,10,16-tétraone;
la 17-propyl-1,14-dihydroxy-12-[2-(4-hydroxy-3-méthoxycyclohexyl)-1-méthyléthyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-propyl-1,14-dihydroxy-12-[2-(4-hydroxy-3-méthoxycyclohexyl)-1-méthyléthyl]-23,25-diméthoxy-13,19,21,27-tétraméthyl-11,28-dioxa-4-azatricyclo[22.3.1.0^{4,9}]octacosane-2,3,10,16-tétraone;
la 17-allyl-1,14,20-trihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-(1-hydroxyprop-2-ényl)-1,14,20-trihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-allyl-1,2-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-3,10,16-trione;
la 17-allyl-1,16-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacosa-14,18-diène-2,3,10-trione;
la 17-propyl-1-hydroxy-12-[2-(3-méthoxy-4-oxocyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-(2,3-dihydroxypropyl)-1,14-dihydroxy-12-[2-(4-hydroxy-3-méthoxycyclohexyl)-1-méthylvinyl]-23,25- diméthoxy-13,19,21,27-tétraméthyl-11,28-dioxa-4-azatricyclo[22.3.1.0^{4,9}]octacos-18-ène-2,3,10,16-tétraone;
la 17-éthanalyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 1,14-dihydroxy-12-[2-(4-hydroxy-3-méthoxycyclohexyl)-1-méthylvinyl]-26,28-diméthoxy-13,22,24,30-tétraméthyl-11,31-dioxa-4,17-diazatétracyclo[25.3.1.0^{4,9}0^{16,20}]hentriaconta-16(20),18,21-triène-2,3,10-trione;
la 1,14-dihydroxy-12-[2-(4-hydroxy-3-méthoxycyclohexyl)-1-méthylvinyl]-26,28-diméthoxy-17-(2-hydroxyéthyl)-13,22,24,30-tétraméthyl-11,31-dioxa-4,17-diazatétracyclo[25.3.1.0^{4,9}0^{16,20}]hentriaconta-16(20),18,21-triène-2,3,10-trione;
la 1,14-dihydroxy-12-[2-(4-hydroxy-3-méthoxycyclohexyl)-1-méthylvinyl]-26,28-diméthoxy-13,22,24,30-tétraméthyl-17-phénylméthyl-11,31-dioxa-4,17-diazatétracyclo[25.3.1.0^{4,9}0^{16,20}]hentriaconta-16(20),18,21-triène-2,3,10-trione;
la 17-allyl-1-hydroxy-12-[2-(3,4-époxycyclohexyl)-1-méthylvinyl]-23,25-diméthoxy-13,19,21,27-tétraméthyl-11,28-dioxa-4-azatricyclo[22.3.1.0^{4,9}]octacosa-14,18-diène-2,3,10,16-tétraone;
le 17-allyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone-C16-oxime;
le 17-allyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone-C16-oxime-O-méthyléther;
la 17-allyl-1,14-dihydroxy-12-[2-(4-(2',5'-dioxahexyloxy)-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-propyl-1-hydroxy-12-[2-(3,4-dihydroxycyclohexyl)-1-méthylvinyl]-23,25-diméthoxy-13,19,21,27-tétraméthy1-11,28-dioxa-4-azatricyclo[22.3.1.0^{4,9}]octacos-18-ène-2,3,10,16-tétraone;
la 1,14-dihydroxy-12-[2-(4-hydroxy-3-méthoxycyclohexyl)-1-méthylvinyl]-23,25-diméthoxy-13,19,21,27-tétraméthyl-17-(2-oxopropyl)-11,28-dioxa-4-azatricyclo[22.3.1.0^{4,9}]octacos-18-ène-2,3,10,16-tétraone;
la 17-allyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azaspiro[tricyclo[22.3.1.0^{4,9}]octacos-18-ène-2,2'-oxiranne]-3,10,16-trione;
la 17-éthanalyl-1,2,14-trihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-3,10,16-trione;
la 1,14-dihydroxy-12-[2-(4-hydroxy-3-méthoxycyclohexyl)-1-méthylvinyl]-26,28-diméthoxy-18-[(phénylséléno)méthyl]-13,22,24,30-tétraméthyl-11,17,31-trioxa-4-azatétracyclo[25.3.1.0^{4,9}.0^{16,20}]hentriaconta-16(20),21-diène-2,3,10-trione;
l'acide benzènesulfonique de l'hydrazide du 4'-méthyl-[17-allyl-1,14-dihydroxy-12-[2-(4-hydroxy-3-méthoxycyclohexyl)-1-méthylvinyl]-23,25-diméthoxy-13,19,21,27-tétraméthy1-11,28-dioxa-4-azatricyclo[22.3.1.0^{4,9}]octacos-18-ène-2,3,10-trione-16-ylidène], ou
un sel pharmaceutiquement acceptable de ceux-ci.

7. La 17-propyl-1-hydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-allyl-1-hydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone, ou
un sel pharmaceutiquement acceptable de celles-ci.

8. Utilisation d'un composé de formule (I) tel que défini à la revendication 1, d'un composé suivant la revendication 7 ou d'un sel pharmaceutiquement acceptable de ceux-ci, comme produit pharmaceutique.

9. Formulation pharmaceutique comprenant un composé de formule (I) tel que défini à la revendication 1, un composé suivant la revendication 7 ou un sel pharmaceutiquement acceptable de ceux-ci, en mélange avec un adjuvant, diluant ou excipient pharmaceutiquement acceptable.

10. Utilisation d'un composé de formule (I) tel que défini à la revendication 1, d'un composé suivant la revendication 7 ou d'un sel pharmaceutiquement acceptable de ceux-ci, dans la préparation d'un médicament à utiliser comme agent immunosuppresseur.

11. Procédé de préparation d'un composé de formule (I) tel que défini à la revendication 1, d'un composé suivant la revendication 7 ou d'un sel pharmaceutiquement acceptable de ceux-ci, qui comprend :
(a) la préparation d'un composé de formule (I), dans lequel un ou plusieurs des [R¹ et R²], [R³ et R⁴] ou [R⁵ et R⁶] représentent deux atomes d'hydrogène vicinaux, par réduction sélective d'un radical [R¹ et R²], [R³ et R⁴] ou [R⁵ et R⁶] correspondant lorsqu'il représente une deuxième liaison entre les deux atomes de carbone vicinaux dans un composé correspondant;
(b) la préparation d'un composé de formule (I) qui contient un ou plusieurs radicaux hydroxyle, par réduction sélective d'un ou de plusieurs radicaux C=O dans un composé correspondant;
(c) la préparation d'un composé de formule (I) qui contient un radical C(OH)=C-C=O, par oxydation sélective d'un radical C(OH)-C-C=O dans un composé correspondant;
(d) la préparation d'un composé de formule (I) qui contient un ou plusieurs radicaux alcoxy, par alcoylation d'un ou de plusieurs radicaux hydroxyle dans un composé correspondant par réaction avec un composé alcoylant approprié;
(e) la préparation d'un composé de formule (I) qui contient un ou plusieurs radicaux hydroxyle, par déprotection d'un ou de plusieurs radicaux hydroxyle protégés dans un composé correspondant, où le groupe protecteur peut être, de préférence, éliminé par hydrogénolyse;
(f) la préparation d'un composé de formule (I) qui contient une double liaison carbone-carbone, par élimination de HL d'un composé correspondant, où L est un groupe partant;
(g) la préparation d'un composé de formule (I) dans lequel Y, R¹⁰ et R²³, avec les atomes de carbone auxquels ils sont fixés, forment un cycle tétrahydrofurannique substitué par un radical CH₂Se(C₆H₅), par réaction d'un halogénure de phénylsélényle avec un composé correspondant dans lequel Y est O et R¹⁰ est allyle;
(h) la préparation d'un composé de formule (I) qui contient un alcool allylique, par oxydation sélective d'un radical allylique dans un composé correspondant;
(i) la préparation d'un composé de formule (I) qui contient un radical cétonique, par oxydation d'un radical hydroxyle dans un composé correspondant;
(j) la préparation d'un composé de formule (I) qui contient un diol vicinal, par oxydation d'une double liaison carbone-carbone dans un composé correspondant;
(k) la préparation d'un composé de formule (I) qui contient un radical aldéhyde, par clivage oxydatif d'un diol vicinal dans un composé correspondant;
(l) la préparation d'un composé de formule (I) dans lequel Y, R¹⁰ et R²³, avec les atomes de carbone auxquels ils sont fixés, forment un cycle pyrrolique, par réaction d'ammoniaque ou d'une amine avec un composé correspondant dans lequel Y est O et R¹⁰ est CH₂CHO;
(m) la préparation d'un composé de formule (I) qui contient un radical époxyde, par cyclisation d'un radical : 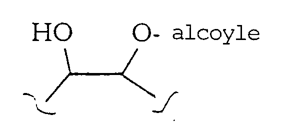 dans un composé correspondant;
(n) la préparation d'un composé de formule (I) dans lequel Y représente un radical oxime, par réaction d'un composé correspondant dans lequel Y est O, avec une amine substituée par un oxygène;
(o) la préparation d'un composé de formule (I) qui contient un radical COCH₃, par oxydation d'un radical alcène terminal dans un composé correspondant;
(p) la préparation d'un composé de formule (I) dans lequel X représente -CH₂O-, par réaction d'un composé correspondant dans lequel X est O, avec du diazométhane, ou
(q) la préparation d'un composé de formule (I) dans lequel Y est une hydrazone ou un dérivé d'hydrazone, par réaction d'un composé correspondant dans lequel Y est O, avec l'hydrazine ou une hydrazine substituée.

## Revendications (Revendications pour l'(les) Etat(s) contractant(s) suivant(s): AT, BE, LU, SE)

1. Composé de formule (I) : 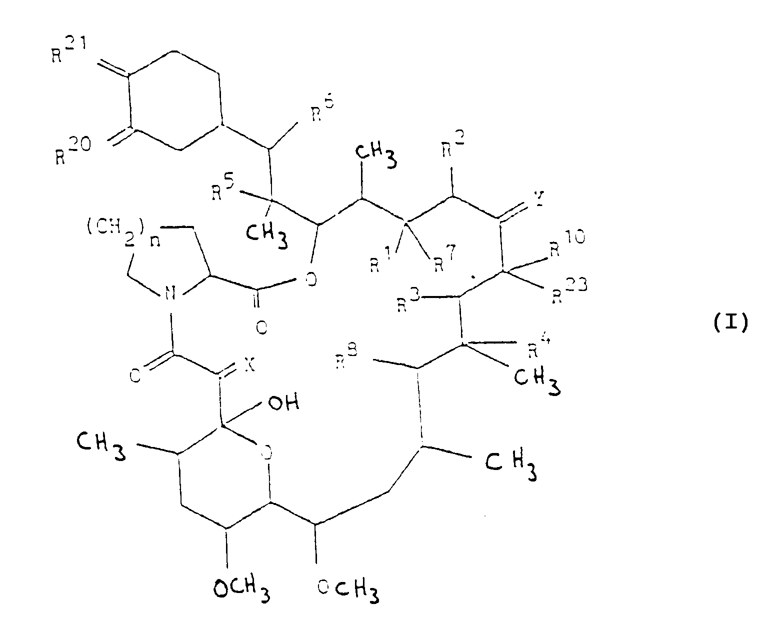 dans lequel :
chaque paire vicinale de substituants [R¹ et R²], [R³ et R⁴] et [R⁵ et R⁶], indépendamment,
a) représente deux atomes d'hydrogène vicinaux, ou
b) forme une deuxième liaison entre les atomes de carbone vicinaux auxquels ils sont fixés;
R⁷ représente H, OH, O-alcoyle en C₁₋₆ ou, en association avec R¹, peut représenter =O;
R⁸ représente H ou OH;
R¹⁰ représente alcoyle en C₁₋₆, alcoyle en C₁₋₆ substitué par un ou plusieurs radicaux hydroxyle, alcényle en C₂₋₆, alcényle en C₂₋₆ substitué par un ou plusieurs radicaux hydroxyle, ou alcoyle en C₁₋₆ substitué par =O;
X représente O, (H,OH) ou -CH₂O-;
Y représente O, (H,OH), N-NR¹¹R¹² ou N-OR¹³;
R¹¹ et R¹², indépendamment, représentent H, alcoyle en C₁₋₆, phényle ou tosyle;
R¹³ représente H ou alcoyle en C₁₋₆;
R²⁰ et R²¹, indépendamment, représentent O, ou ils peuvent représenter, indépendamment, (R²⁰a,H) et (R²¹a,H), respectivement; R²⁰a et R²¹a représentent, indépendamment, OH, O-alcoyle en C₁₋₆ ou OCH₂OCH₂CH₂OCH₃; en outre, R²⁰a et R²¹a peuvent représenter, ensemble, un atome d'oxygène dans un cycle époxyde;
R²³ représente H, et
n est 1 ou 2,
en plus de leurs significations données ci-dessus, Y, R¹⁰ et R²³, avec les atomes de carbone auxquels ils sont fixés, peuvent représenter un hétérocycle à 5 ou 6 membres, contenant N, S ou O, qui peut être saturé ou non saturé et qui peut être substitué par un ou plusieurs radicaux choisis parmi hydroxyle alcoyle en C₁₋₆, alcoyle en C₁₋₆ substitué par un ou plusieurs radicaux hydroxyle, O-alcoyle en C₁₋₆, benzyle et CH₂Se(C₆H₅);
pourvu que :
(a) lorsque [R³ et R⁴] et [R⁵ et R⁶] représentent chacun une liaison carbone-carbone; R⁷ représente H ou un radical OH; R⁸ représente H; R¹⁰ représente méthyle, éthyle, propyle ou allyle; R²⁰a représente OCH₃, et R²¹a représente OH, alors X et Y ne représentent pas O, et
(b) lorsque [R¹ et R²] représente deux atomes d'hydrogène vicinaux; [R³ et R⁴] et [R⁵ et R⁶] représentent chacun une liaison carbone-carbone; R⁷ représente un radical OH; R⁸ représente H; R¹⁰ représente allyle; R²¹a représente OH; X et Y représentent chacun O, et n est 2, alors R²⁰ ne peut pas représenter O ou (OH,H), et
et des sels pharmaceutiquement acceptables de ceux-ci.

2. Composé suivant la revendication 1, dans lequel R¹⁰ représente méthyle, éthyle, propyle ou allyle.

3. Composé suivant la revendication 1 ou 2, dans lequel au moins un des R²⁰a et R²¹a représente OH ou OCH₃.

4. Composé suivant l'une quelconque des revendications précédentes, dans lequel n est 2.

5. Composé suivant l'une quelconque des revendications précédentes, dans lequel R⁷ représente H ou OH.

6. Composé suivant la revendication 1, qui est :
la 17-allyl-1-hydroxy-12-[2-(3,4-diméthoxycyclohexyl)-1-méthylvinyl]-14,23,25-triméthoxy-13,19,21,27-tétraméthyl-11,28-dioxa-4-azatricyclo[22.3.1.0^{4,9}]octacos-18-ène-2,3,10,16-tétraone;
la 17-allyl-1,14-dihydroxy-12-[2-(3,4-diméthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 1,14-dihydroxy-12-[2-(4-hydroxy-3-méthoxycyclohexyl)-1-méthylvinyl]-18-[(phénylséléno)méthyl]-16,26,28-triméthoxy-13,22,24,30-tétraméthyl-11,17,31-trioxa-4-azatétracyclo[25.3.1.0^{4,9}.0^{16,20}]hentriacont-21-ène-2,3,10-trione;
la 17-allyl-1-hydroxy-12-[2-(3,4-diméthoxycyclohexyl)-1-méthylvinyl]-23,25-diméthoxy-13,19,21,27-tétraméthyl-11,28-dioxa-4-azatricyclo[22.3.1.0^{4,9}]octacosa-14,18-diène-2,3,10,16-tétraone;
la 17-allyl-1,2,14-trihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-3,10,16-trione;
la 17-allyl-1,2,14,16-tétrahydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-3,10-dione;
la 17-propyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacosane-2,3,10,16-tétraone;
la 17-propyl-1,14-dihydroxy-12-[2-(4-hydroxy-3-méthoxycyclohexyl)-1-méthyléthyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-propyl-1,14-dihydroxy-12-[2-(4-hydroxy-3-méthoxycyclohexyl)-1-méthyléthyl]-23,25-diméthoxy-13,19,21,27-tétraméthyl-11,28-dioxa-4-azatricyclo[22.3.1.0^{4,9}]octacosane-2,3,10,16-tétraone;
la 17-allyl-1,14,20-trihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-(1-hydroxyprop-2-ényl)-1,14,20-trihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-allyl-1,2-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-3,10,16-trione;
la 17-allyl-1,16-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacosa-14,18-diène-2,3,10-trione;
la 17-propyl-1-hydroxy-12-[2-(3-méthoxy-4-oxocyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-(2,3-dihydroxypropyl)-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-éthanalyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 1,14-dihydroxy-12-[2-(4-hydroxy-3-méthoxycyclohexyl)-1-méthylvinyl]-26,28-diméthoxy-13,22,24,30-tétraméthy1-11,31-dioxa-4,17-diazatétracyclo[25.3.1.0^{4,9}0^{16,20}]hentriaconta-16(20),18,21-triène-2,3,10-trione;
la 1,14-dihydroxy-12-[2-(4-hydroxy-3-méthoxycyclohexyl)-1-méthylvinyl]-26,28-diméthoxy-17-(2-hydroxyéthyl)-13,22,24,30-tétraméthyl-11,31-dioxa-4,17-diazatétracyclo[25.3.1.0^{4,9}0^{16,20}]hentriaconta-16(20),18,21-triène-2,3,10-trione;
la 1,14-dihydroxy-12-[2-(4-hydroxy-3-méthoxycyclohexyl)-1-méthylvinyl]-26,28-diméthoxy-13,22,24,30-tétraméthyl-17-phénylméthyl-11,31-dioxa-4,17-diazatétracyclo[25.3.1.0^{4,9}0^{16,20}]hentriaconta-16(20),18,21-triène-2,3,10-trione;
la 17-allyl-1-hydroxy-12-[2-(3,4-époxycyclohexyl)-1-méthylvinyl]-23,25-diméthoxy-13,19,21,27-tétraméthyl-11,28-dioxa-4-azatricyclo[22.3.1.0^{4,9}]octacosa-14,18-diène-2,3,10,16-tétraone;
le 17-allyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone-C16-oxime;
le 17-allyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone-C16-oxime-O-méthyléther;
la 17-allyl-1,14-dihydroxy-12-[2-(4-(2',5'-dioxahexyloxy)-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-propyl-1-hydroxy-12-[2-(3,4-dihydroxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 1,14-dihydroxy-12-[2-(4-hydroxy-3-méthoxycyclohexyl)-1-méthylvinyl]-23,25-diméthoxy-13,19,21,27-tétraméthyl-17-(2-oxopropyl)-11,28-dioxa-4-azatricyclo[22.3.1.0^{4,9}]octacos-18-ène-2,3,10,16-tétraone;
la 17-allyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azaspiro[tricyclo[22.3.1.0^{4,9}]octacos-18-ène-2,2'-oxiranne]-3,10,16-trione;
la 17-éthanalyl-1,2,14-trihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-3,10,16-trione;
la 1,14-dihydroxy-12-[2-(4-hydroxy-3-méthoxycyclohexyl)-1-méthylvinyl]-26,28-diméthoxy-18-[(phénylséléno)méthyl]-13,22,24,30-tétraméthyl-11,17,31-trioxa-4-azatétracyclo[25.3.1.0^{4,9}.0^{16,20}]hentriaconta-16(20),21-diène-2,3,10-trione;
l'acide benzènesulfonique de l'hydrazide du 4'-méthyl-[17-allyl-1,14-dihydroxy-12-[2-(4-hydroxy-3-méthoxycyclohexyl)-1-méthylvinyl]-23,25-diméthoxy-13,19,21,27-tétraméthy1-11,28-dioxa-4-azatricyclo[22.3.1.0^{4,9}]octacos-18-ène-2,3,10-trione-16-ylidène], ou
un sel pharmaceutiquement acceptable de ceux-ci.

7. La 17-propyl-1-hydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-allyl-1-hydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone, ou
un sel pharmaceutiquement acceptable de celles-ci.

8. Utilisation d'un composé de formule (I) tel que défini à la revendication 1, d'un composé suivant la revendication 7 ou d'un sel pharmaceutiquement acceptable de ceux-ci, comme produit pharmaceutique.

9. Formulation pharmaceutique comprenant un composé de formule (I) tel que défini à la revendication 1, un composé suivant la revendication 7 ou un sel pharmaceutiquement acceptable de ceux-ci, en mélange avec un adjuvant, diluant ou excipient pharmaceutiquement acceptable.

10. Utilisation d'un composé de formule (I) tel que défini à la revendication 1, d'un composé suivant la revendication 7 ou d'un sel pharmaceutiquement acceptable de ceux-ci, dans la préparation d'un médicament à utiliser comme agent immunosuppresseur.

11. Procédé de préparation d'un composé de formule (I) tel que défini à la revendication 1, d'un composé suivant la revendication 7 ou d'un sel pharmaceutiquement acceptable de ceux-ci, qui comprend :
(a) la préparation d'un composé de formule (I), dans lequel un ou plusieurs des [R¹ et R²], [R³ et R⁴] ou [R⁵ et R⁶] représentent deux atomes d'hydrogène vicinaux, par réduction sélective d'un radical [R¹ et R²], [R³ et R⁴] ou [R⁵ et R⁶] correspondant lorsqu'il représente une deuxième liaison entre les deux atomes de carbone vicinaux dans un composé correspondant;
(b) la préparation d'un composé de formule (I) qui contient un ou plusieurs radicaux hydroxyle, par réduction sélective d'un ou de plusieurs radicaux C=O dans un composé correspondant;
(c) la préparation d'un composé de formule (I) qui contient un radical C(OH)=C-C=O, par oxydation sélective d'un radical C(OH)-C-C=O dans un composé correspondant;
(d) la préparation d'un composé de formule (I) qui contient un ou plusieurs radicaux alcoxy, par alcoylation d'un ou de plusieurs radicaux hydroxyle dans un composé correspondant par réaction avec un composé alcoylant approprié;
(e) la préparation d'un composé de formule (I) qui contient un ou plusieurs radicaux hydroxyle, par déprotection d'un ou de plusieurs radicaux hydroxyle protégés dans un composé correspondant, où le groupe protecteur peut être, de préférence, éliminé par hydrogénolyse;
(f) la préparation d'un composé de formule (I) qui contient une double liaison carbone-carbone, par élimination de HL d'un composé correspondant, où L est un groupe partant;
(g) la préparation d'un composé de formule (I) dans lequel Y, R¹⁰ et R²³, avec les atomes de carbone auxquels ils sont fixés, forment un cycle tétrahydrofurannique substitué par un radical CH₂Se(C₆H₅), par réaction d'un halogénure de phénylsélényle avec un composé correspondant dans lequel Y est O et R¹⁰ est allyle;
(h) la préparation d'un composé de formule (I) qui contient un alcool allylique, par oxydation sélective d'un radical allylique dans un composé correspondant;
(i) la préparation d'un composé de formule (I) qui contient un radical cétonique, par oxydation d'un radical hydroxyle dans un composé correspondant;
(j) la préparation d'un composé de formule (() qui contient un diol vicinal, par oxydation d'une double liaison carbone-carbone dans un composé correspondant;
(k) la préparation d'un composé de formule (I) qui contient un radical aldéhyde, par clivage oxydatif d'un diol vicinal dans un composé correspondant;
(l) la préparation d'un composé de formule (I) dans lequel Y, R¹⁰ et R²³, avec les atomes de carbone auxquels ils sont fixés, forment un cycle pyrrolique, par réaction d'ammoniaque ou d'une amine avec un composé correspondant dans lequel Y est O et R¹⁰ est CH₂CHO;
(m) la préparation d'un composé de formule (I) qui contient un radical époxyde, par cyclisation d'un radical : 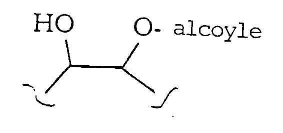 dans un composé correspondant;
(n) la préparation d'un composé de formule (I) dans lequel Y représente un radical oxime, par réaction d'un composé correspondant dans lequel Y est O, avec une amine substituée par un oxygène;
(o) la préparation d'un composé de formule (I) qui contient un radical COCH₃, par oxydation d'un radical alcène terminal dans un composé correspondant;
(p) la préparation d'un composé de formule (I) dans lequel X représente -CH₂O-, par réaction d'un composé correspondant dans lequel X est O, avec du diazométhane, ou
(q) la préparation d'un composé de formule (I) dans lequel Y est une hydrazone ou un dérivé d'hydrazone, par réaction d'un composé correspondant dans lequel Y est O, avec l'hydrazine ou une hydrazine substituée.

## Revendications (Revendications pour l'(les) Etat(s) contractant(s) suivant(s): ES, GR)

1. Procédé de préparation d'un composé de formule (I) : 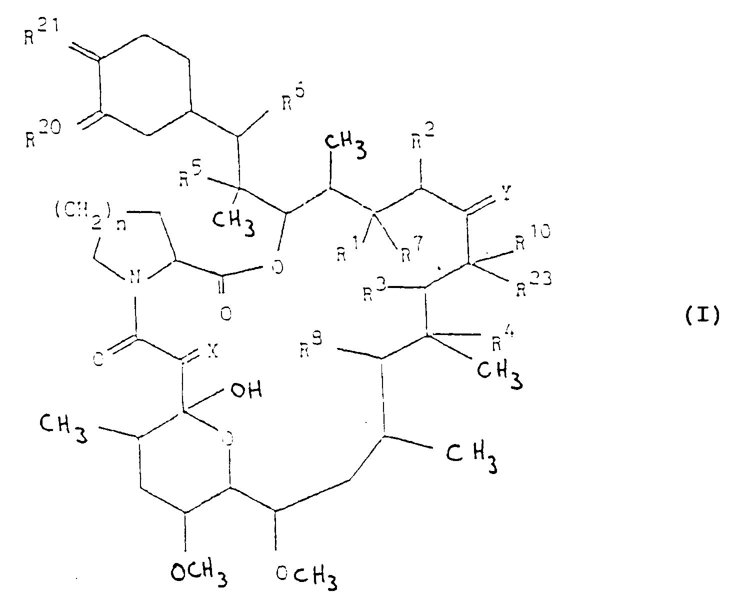 dans lequel :
chaque paire vicinale de substituants [R¹ et R²], [R³ et R⁴] et [R⁵ et R⁶], indépendamment,
a) représente deux atomes d'hydrogène vicinaux, ou
b) forme une deuxième liaison entre les atomes de carbone vicinaux auxquels ils sont fixés;
R⁷ représente H, OH, O-alcoyle en C₁₋₆ ou en association avec R¹, peut représenter =O;
R⁸ représente H ou OH;
R¹⁰ représente alcoyle en C₁₋₆, alcoyle en C₁₋₆ substitué par un ou plusieurs radicaux hydroxyle, alcényle en C₂₋₆, alcényle en C₂₋₆ substitué par un ou plusieurs radicaux hydroxyle, ou alcoyle en C₁₋₆ substitué par =O;
X représente O, (H,OH) ou -CH₂O-;
Y représente O, (H,OH), N-NR¹¹R¹² ou N-OR¹³;
R¹¹ et R¹², indépendamment, représentent H, alcoyle en C₁₋₆, phényle ou tosyle;
R¹³ représente H ou alcoyle en C₁₋₆;
R²⁰ et R²¹, indépendamment, représentent O, ou ils peuvent représenter, indépendamment, (R²⁰a,H) et (R²¹a,H), respectivement; R²⁰a et R²¹a représentent, indépendamment, OH, O-alcoyle en C₁₋₆ ou OCH₂OCH₂CH₂OCH₃; en outre, R²⁰a et R²¹a peuvent représenter, ensemble, un atome d'oxygène dans un cycle époxyde;
R²³ représente H, et
n est 1 ou 2,
en plus de leurs significations données ci-dessus, Y, R¹⁰ et R²³, avec les atomes de carbone auxquels ils sont fixés, peuvent représenter un hétérocycle à 5 ou 6 membres, contenant N, S ou O, qui peut être saturé ou non saturé et qui peut être substitué par un ou plusieurs radicaux choisis parmi hydroxyle alcoyle en C₁₋₆, alcoyle en C₁₋₆ substitué par un ou plusieurs radicaux hydroxyle, O-alcoyle en C₁₋₆, benzyle et CH₂Se(C₆H₅);
pourvu que :
(a) lorsque [R³ et R⁴] et [R⁵ et R⁶] représentent chacun une liaison carbone-carbone; R⁷ représente H ou un radical OH; R⁸ représente H; R¹⁰ représente méthyle, éthyle, propyle ou allyle; R²⁰a représente OCH₃, et R²¹a représente OH, alors X et Y ne représentent pas O, et
(b) lorsque [R¹ et R²] représente deux atomes d'hydrogène vicinaux; [R³ et R⁴] et [R⁵ et R⁶] représentent chacun une liaison carbone-carbone; R⁷ représente un radical OH; R⁸ représente H; R¹⁰ représente allyle; R²¹a représente OH; X et Y représentent chacun O, et n est 2, alors R²⁰ ne peut pas représenter O ou (OH,H), et
et des sels pharmaceutiquement acceptables de ceux-ci, qui comprend :
(a) la préparation d'un composé de formule (I), dans lequel un ou plusieurs des [R¹ et R²], [R³ et R⁴] ou [R⁵ et R⁶] représentent deux atomes d'hydrogène vicinaux, par réduction sélective d'un radical [R¹ et R²], [R³ et R⁴] ou [R⁵ et R⁶] correspondant lorsqu'il représente une deuxième liaison entre les deux atomes de carbone vicinaux dans un composé correspondant;
(b) la préparation d'un composé de formule (I) qui contient un ou plusieurs radicaux hydroxyle, par réduction sélective d'un ou de plusieurs radicaux C=O dans un composé correspondant;
(c) la préparation d'un composé de formule (I) qui contient un radical C(OH)=C-C=O, par oxydation sélective d'un radical C(OH)-C-C=O dans un composé correspondant;
(d) la préparation d'un composé de formule (I) qui contient un ou plusieurs radicaux alcoxy, par alcoylation d'un ou de plusieurs radicaux hydroxyle dans un composé correspondant par réaction avec un composé alcoylant approprié;
(e) la préparation d'un composé de formule (I) qui contient un ou plusieurs radicaux hydroxyle, par déprotection d'un ou de plusieurs radicaux hydroxyle protégés dans un composé correspondant, où le groupe protecteur peut être, de préférence, éliminé par hydrogénolyse;
(f) la préparation d'un composé de formule (I) qui contient une double liaison carbone-carbone, par élimination de HL d'un composé correspondant, où L est un groupe partant;
(g) la préparation d'un composé de formule (I) dans lequel Y, R¹⁰ et R²³, avec les atomes de carbone auxquels ils sont fixés, forment un cycle tétrahydrofurannique substitué par un radical CH₂Se(C₆H₅), par réaction d'un halogénure de phénylsélényle avec un composé correspondant dans lequel Y est O et R¹⁰ est allyle;
(h) la préparation d'un composé de formule (I) qui contient un alcool allylique, par oxydation sélective d'un radical allylique dans un composé correspondant;
(i) la préparation d'un composé de formule (I) qui contient un radical cétonique, par oxydation d'un radical hydroxyle dans un composé correspondant;
(j) la préparation d'un composé de formule (I) qui contient un diol vicinal, par oxydation d'une double liaison carbone-carbone dans un composé correspondant;
(k) la préparation d'un composé de formule (I) qui contient un radical aldéhyde, par clivage oxydatif d'un diol vicinal dans un composé correspondant;
(l) la préparation d'un composé de formule (I) dans lequel Y, R¹⁰ et R²³, avec les atomes de carbone auxquels ils sont fixés, forment un cycle pyrrolique, par réaction d'ammoniaque ou d'une amine avec un composé correspondant dans lequel Y est O et R¹⁰ est CH₂CHO;
(m) la préparation d'un composé de formule (I) qui contient un radical époxyde, par cyclisation d'un radical : 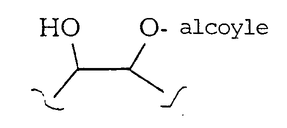 dans un composé correspondant;
(n) la préparation d'un composé de formule (I) dans lequel Y représente un radical oxime, par réaction d'un composé correspondant dans lequel Y est O, avec une amine substituée par un oxygène;
(o) la préparation d'un composé de formule (I) qui contient un radical COCH₃, par oxydation d'un radical alcène terminal dans un composé correspondant;
(p) la préparation d'un composé de formule (I) dans lequel X représente -CH₂O-, par réaction d'un composé correspondant dans lequel X est O, avec du diazométhane, ou
(q) la préparation d'un composé de formule (I) dans lequel Y est une hydrazone ou un dérivé d'hydrazone, par réaction d'un composé correspondant dans lequel Y est O, avec l'hydrazine ou une hydrazine substituée.

2. Procédé suivant la revendication 1, dans lequel R¹⁰ représente méthyle, éthyle, propyle ou allyle.

3. Procédé suivant la revendication 1 ou 2, dans lequel au moins un des R²⁰a et R²¹a représente OH ou OCH₃.

4. Procédé suivant l'une quelconque des revendications précédentes, dans lequel n est 2.

5. Procédé suivant l'une quelconque des revendications précédentes, dans lequel R⁷ représente H ou OH.

6. Procédé suivant la revendication 1, dans lequel le composé de formule (I) est :
la 17-allyl-1-hydroxy-12-[2-(3,4-diméthoxycyclohexyl)-1-méthylvinyl]-14,23,25-triméthoxy-13,19,21,27-tétraméthyl-11,28-dioxa-4-azatricyclo[22.3.1.0^{4,9}]octacos-18-ène-2,3,10,16-tétraone;
la 17-allyl-1,14-dihydroxy-12-[2-(3,4-diméthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 1,14-dihydroxy-12-[2-(4-hydroxy-3-méthoxycyclohexyl)-1-méthylvinyl]-18-[(phénylséléno)méthyl]-16,26,28-triméthoxy-13,22,24,30-tétraméthyl-11,17,31-trioxa-4-azatétracyclo[25.3.1.0^{4,9}.0^{16,20}]hentriacont-21-ène-2,3,10-trione;
la 17-allyl-1-hydroxy-12-[2-(3,4-diméthoxycyclohexyl)-1-méthylvinyl]-23,25-diméthoxy-13,19,21,27-tétraméthyl-11,28-dioxa-4-azatricyclo[22.3.1.0^{4,9}]octacosa-14,18-diène-2,3,10,16-tétraone;
la 17-allyl-1,2,14-trihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-3,10,16-trione;
la 17-allyl-1,2,14,16-tétrahydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-3,10-dione;
la 17-propyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacosane-2,3,10,16-tétraone;
la 17-propyl-1,14-dihydroxy-12-[2-(4-hydroxy-3-méthoxycyclohexyl)-1-méthyléthyl]-23,25-diméthoxy-13, 13,19,21,27-tétraméthyl-11,28-dioxa-4-azatricyclo[22.3.1.0^{4,9}]octacos-18-ène-2,3,10,16-tétraone;
la 17-propyl-1,14-dihydroxy-12-[2-(4-hydroxy-3-méthoxycyclohexyl)-1-méthyléthyl]-23,25-diméthoxy-13,19,21,27-tétraméthyl-11,28-dioxa-4-azatricyclo[22.3.1.0^{4,9}]octacosane-2,3,10,16-tétraone;
la 17-allyl-1,14,20-trihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-(1-hydroxyprop-2-ényl)-1,14,20-trihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-allyl-1,2-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-3,10,16-trione;
la 17-allyl-1,16-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacosa-14,18-diène-2,3,10-trione;
la 17-propyl-1-hydroxy-12-[2-(3-méthoxy-4-oxocyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-(2,3-dihydroxypropyl)-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-éthanalyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 1,14-dihydroxy-12-[2-(4-hydroxy-3-méthoxycyclohexyl)-1-méthylvinyl]-26,28-diméthoxy-13,22,24,30-tétraméthy1-11,31-dioxa-4,17-diazatétracyclo[25.3.1.0^{4,9}0^{16,20}]hentriaconta-16(20),18,21-triène-2,3,10-trione;
la 1,14-dihydroxy-12-[2-(4-hydroxy-3-méthoxycyclohexyl)-1-méthylvinyl]-26,28-diméthoxy-17-(2-hydroxyéthyl)-13,22,24,30-tétraméthyl-11,31-dioxa-4,17-diazatétracyclo[25.3.1.0^{4,9}0^{16,20}]hentriaconta-16(20),18,21-triène-2,3,10-trione;
la 1,14-dihydroxy-12-[2-(4-hydroxy-3-méthoxycyclohexyl)-1-méthylvinyl]-26,28-diméthoxy-13,22,24,30-tétraméthyl-17-phénylméthyl-11,31-dioxa-4,17-diazatétracyclo[25.3.1.0^{4,9}0^{16,20}]hentriaconta-16(20),18,21-triène-2,3,10-trione;
la 17-allyl-1-hydroxy-12-[2-(3,4-époxycyclohexyl)-1-méthylvinyl]-23,25-diméthoxy-13,19,21,27-tétraméthyl-11,28-dioxa-4-azatricyclo[22.3.1.0^{4,9}]octacosa-14,18-diène-2,3,10,16-tétraone;
le 17-allyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone-C16-oxime;
le 17-allyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone-C16-oxime-O-méthyléther;
la 17-allyl-1,14-dihydroxy-12-[2-(4-(2',5'-dioxahexyloxy)-3-méthoxycyclohexyl)-1-méthylvinyl]-23,25- diméthoxy-13,19,21,27-tétraméthyl-11,28-dioxa-4-azatricyclo[22.3.1.0^{4,9}]octacos-18-ène-2,3,10,16-tétraone;
la 17-propyl-1-hydroxy-12-[2-(3,4-dihydroxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 1,14-dihydroxy-12-[2-(4-hydroxy-3-méthoxycyclohexyl)-1-méthylvinyl]-23,25-diméthoxy-13,19,21,27-tétraméthyl-17-(2-oxopropyl)-11,28-dioxa-4-azatricyclo[22.3.1.0^{4,9}]octacos-18-ène-2,3,10,16-tétraone;
la 17-allyl-1,14-dihydroxy-12-[2-(4-hydroxy-3-méthoxycyclohexyl)-1-méthylvinyl]-23,25-diméthoxy-13,19,21,27-tétraméthyl-11,28-dioxa-4-azaspiro[tricyclo[22.3.1.0^{4,9}]octacos-18-ène-2,2'-oxiranne]-3,10,16-trione;
la 17-éthanalyl-1,2,14-trihydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-3,10,16-trione;
la 1,14-dihydroxy-12-[2-(4-hydroxy-3-méthoxycyclohexyl)-1-méthylvinyl]-26,28-diméthoxy-18-[(phénylséléno)méthyl]-13,22,24,30-tétraméthyl-11,17,31-trioxa-4-azatétracyclo[25.3.1.0^{4,9}.0^{16,20}]hentriaconta-16(20),21-diène-2,3,10-trione;
l'acide benzènesulfonique de l'hydrazide du 4'-méthyl-[17-allyl-1,14-dihydroxy-12-[2-(4-hydroxy-3-méthoxycyclohexyl)-1-méthylvinyl]-23,25-diméthoxy-13,19,21,27-tétraméthy1-11,28-dioxa-4-azatricyclo[22.3.1.0^{4,9}]octacos-18-ène-2,3,10-trione-16-ylidène], ou
un sel pharmaceutiquement acceptable de ceux-ci.

7. Procédé de préparation de :
la 17-propyl-1-hydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone;
la 17-allyl-1-hydroxy-12-[2-(4-hydroxy-3-méthoxycyclohexyl)-1-méthylvinyl]-23,25-diméthoxy-13,19,21,27-tétraméthyl-11,28-dioxa-4-azatricyclo[22.3.1.0^{4,9}]octacos-18-ène-2,3,10,16-tétraone, ou un sel pharmaceutiquement acceptable de celles-ci, qui comprend la réduction sélective d'un composé correspondant ayant une double liaison carbone-carbone en position 14.

8. Procédé de préparation d'une composition pharmaceutique comprenant comme ingrédient actif, un composé de formule (I) tel que défini à la revendication 1, un composé suivant la revendication 7 ou un sel pharmaceutiquement acceptable de ceux-ci, qui comprend le mélange de l'ingrédient actif avec un adjuvant, diluant ou excipient pharmaceutiquement acceptable.
